# DEMANDE DE BREVET EUROPEEN

(11) **EP 4 666 998 A1**
(43) Date de publication de la demande: **24.12.2025**
(21) Numéro de dépôt: 24305990.4
(22) Date de dépôt: 21.06.2024
(51) Int. Cl.: A61K 9/00, A61K 47/34, A61K 47/36, A61K 47/50, C12N 5/00

(54) **COMPOSITION COMPRENANT UN POLYMERE BIOCOMPATIBLE ET AU MOINS UNE VESICULE EXTRACELLULAIRE ET PROCEDE DE PREPARATION**

(71) Demandeur: Organes Tissus Régénération Réparation Remplacement, 75001 Paris (FR); Barritault, Denis, 75001 Paris (FR); Université de Caen Normandie, 14000 Caen (FR); Centre National de la Recherche Scientifique, 75016 Paris (FR)
(72) Inventeur: BARRITAULT, Denis, 75001 Paris (FR); CHIAPPINI, Franck, 94200 Ivry Sur Seine (FR); RENOULT, Charlène, 35580 Guichen (FR); BERNAUDIN, Myriam, 14990 Bernières sur mer (FR); OMAR, Touzani, 14310 Villy-Bocage (FR); COLLOC'H, Nathalie, 14112 Bieville-Beuville (FR)
(74) Mandataire: Novagraaf Technologies

(57) **Abrégé**

La présente invention se rapporte à une composition pharmaceutique comprenant un polymère biocompatible de formule générale (I) AaXxYy ou (II) AaXxYyZz, et au moins une vésicule extracellulaire. La présente invention se rapporte également à un procédé de préparation de vésicules extracellulaires.

La présente invention trouve une application notamment dans les domaines thérapeutiques, pharmaceutiques et vétérinaires.

## Description

### Domaine technique

La présente invention se rapporte à une composition pharmaceutique comprenant un polymère biocompatible de formule générale (I) AaXxYy et au moins une vésicule extracellulaire.

La présente invention se rapporte également à une composition pharmaceutique comprenant un polymère biocompatible de formule générale (II) AaXxYyZz et au moins une vésicule extracellulaire.

La présente invention se rapporte également à un procédé de préparation de vésicules extracellulaires et aux vésicules obtenues par ledit procédé.

La présente invention trouve une application notamment dans les domaines prophylactiques, thérapeutiques, pharmaceutiques humains et vétérinaires.

Dans la description ci-dessous, les références entre crochets [ ] renvoient à la liste des références présentée à la fin du texte.

### Etat de la technique

L'implantation de cellules, de tissus ou d'organes à des fins thérapeutiques, notamment le traitement de lésions tissulaires est un enjeu majeur de la médecine.

Toutefois, après administration des cellules, les rendements concernant leur intégration et/ou l'efficacité thérapeutique au vu du nombre de cellules administrées reste très faible, impliquant ainsi la nécessité de répétition du traitement et des coûts de traitement très importants. En outre, les faibles rendements et coûts importants limitent les pathologies et/ou les patients susceptibles d'être traités par ces procédés.

Il existe dans l'état de la technique des composés susceptibles d'améliorer l'environnement tissulaire. Par exemple, des agents de Régénération tissulaires ou RGTA@ sont connus ([1], US8790631). Ces composés sont notamment connus pour leurs utilisation seul ou en association, par exemple avec de l'acide Hyaluronique ([2], WO2020151900), par exemple dans le traitement de lésions tissulaires, telles que les lésions cutanées. Les agents de Régénération tissulaires ou RGTA^{®} sont par exemple connus pour être utiles dans le traitement de lésions tissulaires de nature et d'étiologie différentes ainsi que d'origines multiples tant chez l'homme qu'en médecine vétérinaire et dans des modèles animaux, par exemple équin, ex-vivo, et in vitro ([3], Barritault, et al., 10.1007/s10719-016-9744-5, 4], Barritault, et al., 10.1016/j.jbspin.2016.06.012). L'utilisation des agents de Régénération tissulaires ou RGTA@ avec des cellules progénitrices exogènes, par exemple des cellules souches d'origines diverses comme les Cellules Souches Mésenchymateuses (MSC) est également connus ([5], EP3302523B1).

Il est également connu que des RGTA@ peuvent être utiles dans le traitement de lésions tissulaires via un remplacement des héparanes sulfates endogènes détruits uniquement sur le site de la lésion ([6], Meddahi, et al., 10.1002/jbm.10283), ([7], Pereira, et al., 10.1007/s10719-022-10047-x) Toutefois, après administration, les rendements concernant l'intégration des cellules et/ou le ciblage de la lésion à traiter reste faible et est susceptible d'impliquer une éventuelle répétition du traitement et également des coûts de traitement importants. En outre, les rendements et coûts sont susceptibles de limiter les pathologies et/ou les patients susceptibles d'être traités par ces procédés ([3], Barritault, et al., 10.1007/s10719-016-9744-5, 4], Barritault, et al., 10.1016/j.jbspin.2016.06.012).

Il existe donc un réel besoin dans l'état de la technique de trouver un composé et/ou une composition permettant d'améliorer le traitement de lésions tissulaires.

L'utilisation de Vésicules Extracellulaires (VE) pour le traitement de lésions tissulaires est également connu, les vésicules extracellulaires étant, par rapport aux cellules, préparées et stockées en large quantité pour une utilisation au plus grand nombre de patients ([8], Xin, et al., 10.1038/jcbfm.2013.152) ([9], Doeppner, et al., 10.5966/sctm.2015-0078) ([10], Jeyaram, et al., 10.1208/s12248-017-0160-y) ([11], Ma, et al., 10.4252/wjsc.v12.i8.814).

Les vésicules extracellulaires sont des nanoparticules sécrétées renfermant une membrane lipidique et liées à des phospholipides qui transportent un complément de lipides, d'ADN, d'ARN, de protéines, de métabolites et de glycanes qui reflètent l'identité et l'état moléculaire de leur cellule d'origine. La chimie de la surface des vésicules extracellulaires a une grande importance biologique pour la régulation de l'orientation, du ciblage et de l'absorption des vésicules extracellulaires par les cellules réceptrices ([12], Yanez-Mo, et al., 10.3402/jev.v4.27066) ([13], Hallal, et al., 10.1002/jev2.12260),([14], Buzas, et al., 10.1007/s00281-018-0682-0).

Toutefois, après administration, les rendements concernant l'intégration des vésicules extracellulaires et/ou le ciblage de la lésion à traiter reste également faible et est susceptible d'impliquer une éventuelle répétition d'administrations et des coûts de traitements importants. En outre, les rendements et coûts sont susceptibles de limiter les pathologies et/ou les patients susceptibles d'être traités par ces procédés ([15], Kalimuthu, et al., 10.1186/s13036-019-0160-9),([16], Uddin, et al., 10.1016/j.ipha.2024.02.004).

Il existe donc un réel besoin dans l'état de la technique de trouver un composé et/ou une composition permettant d'améliorer le traitement de lésions tissulaires.

Les traitements connus de lésions tissulaire peuvent présenter des efficacités relatives.

Il existe donc un réel besoin dans l'état de la technique de trouver un composé et/ou une composition permettant d'améliorer l'efficacité et/ou réduire les coûts liés au traitement de lésions tissulaires.

### Description de l'invention

La présente invention a précisément pour but de répondre à ces besoins en fournissant une composition pharmaceutique comprenant
- un polymère biocompatible de formule générale (I) suivante

   AaXxYy (I)

   dans laquelle :
   A représente un monomère,
   X représente un groupement R₁COOR₂ ou -R₉(C=O)R₁₀,
   Y représente un groupement O ou N-sulfonate et répondant à l'une des formules suivante -R₃OSO₃R₄, -R₅NSO₃R₆, -R₇SO₃R₈ ou R₇SO₃R₈ dans lesquelles :
      R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation M⁺,
      R₇ et R₁₀ représente indépendamment une liaison, une chaîne hydrocarbonée aliphatique, éventuellement ramifiée et/ou insaturée,
      a représente le nombre de monomères,
      x représente le taux de substitution des monomères A par des groupements X,
      y représente le taux de substitution des monomères A par des groupements Y, et
- au moins une vésicule extracellulaire.

Avantageusement, les inventeurs ont démontré de manière surprenante que la composition comprenant un polymère biocompatible et au moins une vésicule extracellulaire selon l'invention permet avantageusement de traiter des lésions tissulaires.

Avantageusement, les inventeurs ont démontré de manière surprenante que les polymères biocompatibles selon l'invention, par exemple des RGTA@, peuvent avantageusement cibler des sites lésionnaires, par exemple des lésions tissulaires, également désignés sites de lésions, est permettre ainsi à au moins une vésicule extracellulaire ou à des vésicules extracellulaires d'être avantageusement adressées et séquestrées sur le site de la lésion à traiter, à réparer. Avantageusement, les inventeurs ont démontré que le ciblage et/ou adressage et/ou séquestration au niveau de la lésion tissulaire permet un meilleur traitement de la lésion, par exemple une accélération de la cicatrisation et/ou de la régénération tissulaire tout en restaurant la fonction tissulaire. En d'autres termes, la composition selon l'invention permet avantageusement une accélération de la cicatrisation et/ou de la régénération tissulaire et une restauration de la fonctionnalité tissulaire.

En outre, les inventeurs ont démontré de manière surprenante et inattendue que la composition comprenant un polymère biocompatible et au moins une vésicule extracellulaire selon l'invention permet avantageusement une réparation rapide et fonctionnelle des lésions tissulaires. En particulier, les inventeurs sont les premiers à avoir démontré de manière surprenante qu'après administration la composition comprenant un polymère biocompatible et au moins une vésicule extracellulaire selon l'invention est spécifiquement adressée, notamment via le polymère biocompatible, au site de la lésion tissulaire. En d'autres termes, la composition selon l'invention est spécifiquement adressée au site de la lésion tissulaire permettant avantageusement d'accélérer la réparation de la lésion tissulaire et/ou de favoriser la reconstruction du site lésionnel. La composition selon l'invention permet en outre avantageusement et de manière surprenante un traitement rapide et optimum de la lésion.

Les inventeurs ont également démontré que la composition selon l'invention permet avantageusement et de manière surprenante d'augmenter le recrutement cellulaire sur le site de la lésion. La composition selon l'invention permet, notamment via une augmentation du recrutement cellulaire sur le site de la lésion, d'accélérer la réparation de la lésion tissulaire et/ou de favoriser la reconstruction du site lésionnel. La composition comprenant un polymère biocompatible et au moins une vésicule extracellulaire selon l'invention permet avantageusement l'obtention d'un effet thérapeutique identique quel que soit le site et/ou mode d'administration. Par exemple, la composition comprenant un polymère biocompatible et au moins une vésicule extracellulaire selon l'invention peut être administrée à distance comme à proximité du site lésionnel sans modification de ces effets concernant le traitement de la lésion et la régénération tissulaire.

En d'autres termes, les inventeurs ont également démontré que la composition selon l'invention, après administration, cible la lésion tissulaire et permet avantageusement de traiter ladite lésion par recrutement et différenciation des cellules présentent au niveau de la lésion permettant une cicatrisation de la lésion.

Les inventeurs ont également démontré que la composition selon l'invention permet avantageusement une restauration microenvironnement cellulaire et matriciel, notamment des éléments protéiques et glycaniques de la matrice extracellulaire au niveau de la lésion, notamment via une stimulation de la néosynthèse et remodelage de la matrice extracellulaire au niveau de la lésion.

Les inventeurs sont les premiers à avoir démontré que les polymères biocompatibles selon l'invention se fixent sur les vésicules extracellulaires, par exemple via des peptides et/ou des protéines d'adhésions cellulaires, des peptides et/ou des protéines transmembranaires, des peptides et/ou des protéines extra-membranaires, des peptides et/ou des protéines sécrétées, et/ou des lipoprotéines membranaires, naturelle ou synthétique, par exemple des peptides et/ou les protéines synthétiques, par exemple tel que décrits dans Fields, 10.1002/0471140864.ps1801 s26 [17] avec une affinité très forte.

Les inventeurs ont également démontré de manière surprenante et inattendue que la combinaison de polymères biocompatibles et des vésicules extracellulaires, par exemple la fixation de polymères biocompatible sur des vésicules extracellulaires, permet de manière surprenante un adressage des vésicules extracellulaires jusqu'à la lésion tissulaire et/ou le site lésionnel.

Les inventeurs ont également démontré de manière surprenante et inattendue que la combinaison des polymères biocompatibles et des vésicules extracellulaires selon l'invention permet avantageusement et de manière surprenante un ancrage de la combinaison des polymères biocompatibles et des vésicules extracellulaires dans la matrice du tissu lésé. En outre, les inventeurs ont démontré, de manière surprenante, que l'ancrage peut avantageusement stimuler localement les mécanismes biologiques permettant une reconstruction du tissu lésé.

Dans la présente par monomère on entend par exemple un monomère choisi dans le groupe comprenant les sucres, les esters, les alcools, les acides aminés ou les nucléotides ou des dérivés de ceux-ci.

Dans la présente invention, les monomères A constituent les éléments de base des polymères de formule I peuvent être identiques ou différents.

Dans la présente invention, les monomères A identiques ou différents peuvent être indépendamment choisis parmi les sucres ou dérivés de ceux-ci.

Dans la présente invention, les monomères A peuvent être indépendamment des monomères de formule suivante : 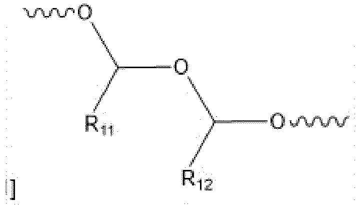 dans laquelle R₁₁ et R₁₂ représente indépendamment un atome d'oxygène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué, un groupement de formule -R₁₃-(X)n-R₁₄ dans laquelle R₁₃ représente une chaine carbonée aliphatique en C₁ à C₄, éventuellement ramifiée et/ou insaturée, X représente un hétéroatome choisi parmi l'oxygène et l'azote, est un entier compris de 1 à 4 et R₁₄ est un atome d'hydrogène, une chaîne hydrocarbonée aliphatique, éventuellement ramifiée et/ou insaturée, un groupe hétéroaryle comprenant indépendamment un ou plusieurs atomes d'oxygène et/ou d'azote, une fonction aldéhyde, un groupe acide carboxylique, un diol, un diol substitué.

Dans la présente invention, l'association de monomères peut permettre de former un squelette polymérique, par exemple un squelette polymérique de nature polyester, polyalcool, polysaccharidique, du type des acides nucléiques ou des protéines.

Dans la présente invention, parmi les polyesters, il peut s'agir par exemple de copolymères de biosynthèse ou synthèse chimique, par exemple des polyesters aliphatiques ou d'origine naturelle par exemple les polyhydroxyalconaotes.

Dans la présente invention, les polysaccharides et leurs dérivés peuvent être d'origine bactérienne, fongique, animale et/ou d'origine végétale. Il peut s'agir par exemple de polysaccharides à chaîne simple, par exemple les poly-glucoses, par exemple le dextran, la cellulose, le bêta glucan, amidon/glycogène ou d'autres monomères comprenant des unités plus complexes, par exemple les xanthanes, par exemple le glucose, mannose, le galactose, les acides uroniques et l'acide glucuronique, ou les glucuronanes, et glucoglucuronane, les galactosamines, l'acide iduronique et les dérivés protéiques fortement glycosylés, par exemple les mucines.

Dans la présente invention, les polysaccharides d'origine végétale peuvent être à simple chaîne, par exemple la cellulose (glucose), l'hémicellulose, les pectines (acide galacturonique), les fucanes, l'amidon ou plus complexe comme les alginates (acide galuronique et mannuronique).

Dans la présente invention, les polysaccharides d'origine fungique peuvent être par exemple le stéroglucane.

Dans la présente invention, les polysaccharides d'origine animale peuvent être par exemple les chitines ou le chitosan (glucosamine).

Dans la présente invention, les monomères A constituant les éléments de base des polymères de formule I peuvent être avantageusement identiques.

Dans la présente invention, les monomères A constituant les éléments de base des polymères de formule I peuvent être avantageusement le glucose.

Le nombre de monomères A défini dans la formule (I) par « a » peut-être tel que la masse desdits polymères de formule (I) est supérieure ou égale à 1000 daltons, par exemple supérieure à 2000 daltons. Le nombre de monomères A défini dans la formule (I) par « a » peut-être tel que la masse desdits polymères de formule (I) est environ entre 1 000 et 6 000 daltons, par exemple ce qui correspond à au moins 5 monomères de glucose. Par exemple la masse desdits polymères de formule (I) peut environ être entre 3000 daltons et 6000 daltons, par exemple ce qui correspond à 12 à 20 monomères de glucose.

Le nombre de monomères A défini dans la formule (I) par « a » peut-être également tel que la masse desdits polymères de formule (I) est inférieure à environ 2 500 000 daltons, par exemple ce qui correspond à 7000 monomères de glucose.

De façon avantageuse, la masse desdits polymères de formule (I) peut être comprise de 3000 à 250 000 daltons, par exemple de 3000 à 6000 daltons, ou par exemple de 20 000 à 250 000 daltons, ou par exemple de 75 000 à 150 000 daltons.

Dans la présente invention, dans le groupement -R₁COOR₂ représentant X, R₁ peut être un alkyle en C₁ à C₆, par exemple un méthyl, un éthyl, un butyl, un propyl, un pentyl, de préférence un groupement méthyl, et R₂ peut être une liaison, un alkyle en C₁ à C₆, par exemple un méthyl, un éthyl, un butyl, un propyl, un pentyl, un groupement R₂₁R₂₂ dans lequel R₂₁ est un anion et R₂₂ un cation choisi dans le groupe des métaux alcalins.

De préférence, le groupement X est le groupement de formule - R₁COOR₂ dans laquelle R₁ est un groupement méthyle -CH₂- et R₂ un groupement R₂₁R₂₂ dans lequel R₂₁ est un anion et R₂₂ un cation choisi dans le groupe des métaux alcalins, de préférence le groupement X est un groupement de formule -CH₂-COO⁻ ou carboxyméthyl.

Dans la présente invention, dans le groupement -R₉(C=O)R₁₀ représentant X, R₉ peut être un alkyle en C₁ à C₆, par exemple un méthyl, un éthyl, un butyl, un propyl, un pentyl, de préférence un groupement méthyl, et R₁₀ peut être une liaison, un alkyle en C₁ à C₆, par exemple un méthyl, un éthyl, un butyl, un propyl, un pentyl, un hexyl.

Le taux de substitution de l'ensemble des monomères A par les groupements X défini dans la formule générale (I) par « x » peut être compris de 10 à 150%, de 40 à 80%, et de préférence de l'ordre de 50% ou 60%.

Dans la présente invention, dans le groupement répondant à l'une des formules suivantes -R₃OSO₃R₄, -R₅NSO₃R₆, -R₇SO₃R₈ ou R₇SO₃R₈ et représentant le groupement Y, R₃ peut être une liaison, un alkyle en C₁ à C₆, par exemple un méthyl, un éthyl, un butyl, un propyl, un pentyl, de préférence un groupement méthyl, R₅ peut être une liaison, un alkyle en C₁ à C₆, par exemple un méthyl, un éthyl, un butyl, un propyl, un pentyl, de préférence un groupement méthyl, R₇ peut être une liaison, un alkyle en C₁ à C₆, par exemple un méthyl, un éthyl, un butyl, un propyl, un pentyl, de préférence un groupement méthyl, R₄, R₆ et R₈ peuvent être indépendamment un atome d'hydrogène ou un cation M⁺, par exemple M⁺ peut être un métal alcalin.

De préférence, le groupement Y est le groupement de formule R₇SO₃R₈ dans lequel R₇ est une liaison et R₈ est un métal alcalin choisi dans le groupe comprenant le lithium, le sodium, le potassium, le rubidium et le césium. De préférence, le groupement Y est un groupement -SO₃⁻, -SO₃⁻ Na⁺

Le taux de substitution de l'ensemble des monomères A par les groupements Y défini dans la formule générale (I) par « y » peut être compris de 10 à 170%, de 30 à 150%, de 55 à 160%, de 55 à 85%, de 120 à 160%, et de préférence de l'ordre de 70, 140 ou 150%.

Dans la présente invention, la définition des taux de substitutions ci-dessus, on entend par un taux de substitution « x » de 100%, le fait que chaque monomère A du polymère de l'invention contient statistiquement un groupement X. De même, on entend par un taux de substitution « y » de 100%, le fait que chaque monomère du polymère de l'invention contient statistiquement un groupement Y. Les taux de substitution supérieurs à 100% traduisent le fait que chaque monomère porte statistiquement plus d'un groupement du type considéré ; à l'inverse, les taux de substitution inférieurs à 100% traduisent le fait que chaque monomère porte statistiquement moins d'un groupement du type considéré.

Les polymères peuvent également comprendre des groupements chimiques fonctionnels, désignés Z, différents de X et Y.

Dans la présente invention, les groupements Z peuvent être identiques ou différent, et peuvent indépendamment être choisis dans le groupe comprenant des acides aminés, des acides gras des alcools gras, des céramides, ou des dérivés de ceux-ci, ou des séquences nucléotidiques d'adressages, des anticorps, des fragments d'anticorps.

Les groupements Z peuvent également représenter des agents actifs identiques ou différents. Il peut s'agir par exemple d'agents thérapeutiques, d'agents de diagnostic, d'un anti-inflammatoire, d'un antimicrobien, d'un antibiotique, d'un agent antiviral, d'un facteur de croissance, d'une cytokine de communication cellulaire, par exemple un interféron, d'une enzyme, d'un composé antioxydant, polyphénols, des tanins, des anthocyanes, des lycopènes, des terpénoïdes et des stilbènes par exemple le resvératrol. Dans la présente invention, le groupement Z peut être avantageusement un acide gras saturé ou insaturé, ou du cholestérol ou de l'un de ces dérivés. Il peut s'agir par exemple d'un dérivé de cholestérol choisi dans le groupe comprenant le cholécalciférol, la cortisone, le cortisol, les hormones sexuelles et leurs dérivés. Il peut s'agir par exemple d'un acide gras choisi dans le groupe comprenant l'acide acétique, l'acide caprylique, l'acide caprique, l'acide laurique, l'acide myristique, l'acide palmitique, l'acide stéarique, l'acide arachidique, l'acide béhénique, l'acide lignocérique, l'acide cérotique, l'acide myristoléique, l'acide palmitoléique, l'acide sapiénique, l'acide oléique, l'acide élaïdique, l'acide trans-vaccénique, l'acide linoléique, l'acide linolélaïdique, l'acide α-linolénique, l'acide γ-linolénique, l'acide dihomo-γ-linolénique, l'acide arachidonique, l'acide eicosapentaénoïque, l'acide clupanodonique ou l'acide docosahexaénoïque. Il peut s'agir d'un eicosanoïde, par exemple choisi dans le groupe comprenant les leucotriènes, les prostanoïdes, les prostaglandines, les thromboxanes et les prostacyclines. De préférence, l'acide gras est l'acide acétique.

Dans la présente invention, le groupement Z peut être avantageusement un acide aminé de la série L ou D choisi dans le groupe comprenant l'alanine, l'asparagine, une chaine aromatique par exemple la tyrosine, la phénylalanine, le tryptophane. De préférence, l'acide aminé est la phénylalanine.

Dans la présente invention, le groupement Z peut être un antioxydant, par exemple la vitamine A, C, E, B9, B6, le glutathion, le sélénium, les polyphénols, par exemple les catéchines, par exemple du thé vert, les flavonoïdes, les tanins, les anthocyanes, par exemple des fruits rouges, les lycopènes, les terpénoïdes et le resvératrol.

Dans la présente invention, le groupement Z peut être des composés anti-âges, par exemple des rétinoïdes, des allantoïnes.

Dans la présente invention, le groupement Z peut être des anticorps, des fragments d'anticorps, par exemple des fragments Fab. Il peut s'agir par exemple d'anticorps et/ou de fragments d'anticorps d'adressages.

Avantageusement les groupements Z peuvent conférer aux polymères des propriétés biologiques ou physicochimiques supplémentaires. Par exemple les groupements Z peuvent augmenter la solubilité ou la lipophilie dudit polymère permettant par exemple une meilleure diffusion ou pénétration tissulaire.

Avantageusement, les groupements Z peuvent conférer aux polymères des propriétés biologiques ou physicochimiques supplémentaires. Ainsi, les polymères de l'invention, par exemple lorsque le groupement Z est choisi parmi un composé antioxydant, un composé anti-âge, les polymères de l'invention peuvent avantageusement véhiculer ces composés et ainsi fournir un effet biologique additionnel et/ou complémentaire.

Des polymères dans lesquels Z est présent peuvent répondre à la formule II suivante : Aa Xx Yy Zz (II) dans laquelle, A, X, Y, a, x, y sont tel que défini ci-dessus et z représente le taux de substitution par des groupements Z.

Dans la présente invention le taux de substitution par des groupements Z représenté par « z » peut être compris de 1 à 50%, de 10 à 25%, de préférence égale à 15, 20 ou 25%.

Les groupements X, Y et Z peuvent être indépendamment fixés sur le monomère A et/ou indépendamment fixés les uns aux autres. Lorsqu'au moins un des groupements X, Y et Z est indépendamment fixé sur un groupement X, Y et Z différent du premier, un desdits groupements X, Y ou Z est fixé au monomère A.

Ainsi, les groupements Z peuvent être fixés par covalence directement sur les monomères A ou fixés par covalence sur les groupements X et/ou Y.

Dans la présente invention les groupements Z peuvent aussi être conjugués aux polymères de formule AaXxYy par des liaisons autres que covalentes, par exemple par des liaisons ioniques, par exemple via des interactions ioniques, des liaisons hydrophiles ou des liaisons hydrophobes. Les polymères de l'invention peuvent alors constituer un système de vectorisation de Z.

Dans la présente invention, le polymère peut être par exemple un polymère choisi dans le groupe comprenant les composés OTR4120, OTR41201, OTR41202, OTR41203, OTR41205, OTR41210 OTR41301, OTR41302, OTR41303, OTR41305, OTR41310, OTR3131, OTR4131, OTR4132, de préférence OTR4132.

Dans la présente invention, le polymère peut être par exemple un polymère choisi dans le groupe comprenant les composés OTR41201, OTR41202, OTR41203, OTR41205, OTR41210, OTR4120, OTR4122, OTR4125, OTR41301, OTR41302, OTR41303, OTR41305, OTR41310, OTR3131, OTR4131, OTR4132, OTR4135, OTR415 avec les caractéristiques mentionnées dans le tableau 1 ci-dessous.

**Tableau 1 : liste et caractéristiques de polymères. (*ou phénylalanine méthyl ester).**

| **Polymère** | | **A : glucose** | **X -CH₂COO** | **Y -SO₃⁻** | **Z -OCCH₃** | **Z* Phénylalanine** |
|---|---|---|---|---|---|---|
| **Nom du RGTA** | **Poids moléculaire moyen +/-15%** | **Polymère de Dextran de départ (PM en Dalton)** | **% de substitutio n CM /glucose** | **% de substitution S0₄ / glucose)** | **% de substitution OCCH₃ /glucose** | |
| CMDS OTR4120 1 | 3 000 | 1 500 | 60+/-20 | 150+/-20 | 0 | |
| CMDS OTR4120 2 | 6 000 | 3 000 | 60+/-20 | 150+1-20 | 0 | |
| CMDS OTR4120 3 | 10 000 | 5 000 | 60+/-20 | 150+1-20 | 0 | |
| CMDS OTR4120 5 | 20 000 | 10 000 | 60+/-20 | 150+/-20 | 0 | |
| CMDS OTR4121 0 | 40 000 | 20 000 | 60+/-20 | 150+/-20 | 0 | |
| CMDS OTR4120 | 80 000 | 40 000 | 60+/-20 | 150+/-20 | 0 | |
| CMDS OTR4122 | 220 000 | 110 000 | 60+/-20 | 150+1-20 | 0 | |
| CMDS OTR4125 | 500 000 | 250 000 | 60+/-20 | 150+1-20 | 0 | |
| CMDSA OTR4130 1 | 3 000 | 1 500 | 60+/-20 | 140+1-20 | 20+/-5 | |
| CMDSA OTR4130 2 | 6 000 | 3 000 | 60+/-20 | 140+1-20 | 20+/-5 | |
| CMDSA OTR4130 3 | 10 000 | 5 000 | 60+/-20 | 140+/-20 | 20+/-5 | |
| CMDSA OTR4130 5 | 20 000 | 10 000 | 60+/-20 | 140+1-20 | 20+/-5 | |
| CMDSA OTR4131 0 | 40 000 | 20 000 | 60+/-20 | 140+1-20 | 20+/-5 | |
| CMDSA OTR4131 | 80 000 | 40 000 | 60+/-20 | 140+1-20 | 20+/-5 | |
| CMDSA OTR4132 | 220 000 | 110 000 | 60+/-20 | 140+1-20 | 20+/-5 | |
| CMDSA OTR4135 | 500 000 | 250 000 | 60+/-20 | 140+1-20 | 20+/-5 | |
| CMDSP OTR415 | 5000 | | 60+/-20 | 70+/-15 | - | 15+/-5 |

| | | | | | | |
|---|---|---|---|---|---|---|
| Tableau 1 : Polymères des familles Aa Xx Yy (I) et Aa Xx Yy Zz (II) dans lesquels A est le glucose (PM 180 Da), X est CarboxyMéthyl (PM 58 Da) Y : SO₃⁻ (PM 80 Da) et Z est Acétate (PM 43 Da) ou phénylalanine (PM 165 Da). | | | | | | |

Dans la présente invention, la composition peut comprendre une concentration de 0,001 à 30000 µg/mL en poids de polymère biocompatible, par exemple de 0,1 to 300 µg/mL en poids de polymère biocompatible, par rapport au volume de la composition. Par exemple la composition peut comprendre une concentration de 1 à 300 µg/mL, de 10 à 300 µg/ml, de 20 à 200 µg/mL, par exemple de 1 à 10 µg/mL en poids de polymère biocompatible par rapport au volume total de la composition.

Dans la présente invention, la composition peut être formulée et/ou adaptée selon son administration. Par exemple, pour une administration par voie parentérale la composition peut être administrée afin de délivrer une dose de polymère biocompatible comprise de 0,01 à 5 mg par kilogramme (kg) de poids corporel (mg/kg), de préférence de 0,1 à 1,5 mg par kilogramme de poids corporel. Par exemple la fréquence d'administration de la composition peut être, par exemple une administration par jour, tous les deux jours ou tous les trois jours, par exemple 2 à 3 fois par semaine. Par exemple la fréquence d'administration de la composition peut être, par exemple une administration par 24 heures, par 48 heures ou par 72 heures, par exemple 2 à 3 fois par semaine.

Par exemple, pour une administration par voie orale la composition peut être administrée afin de délivrer une dose de polymère biocompatible comprise de 0,1 à 5 mg par kilogramme de poids corporel, de préférence de 0,01 à 1,5 mg/kg à la fréquence d'une administration quotidienne ou bi hebdomadaire.

Pour une administration sublinguale la prise peut être quotidienne ou bi hebdomadaire et comprise entre 0,5 µg/kg et 100 µg/kg de poids corporel.

Par exemple, pour une administration intra-artérielle, le polymère biocompatible peut être à une concentration comprise de 0,01 à 400 µg/mL en poids de polymère biocompatible par rapport au volume total de la composition, de préférence de 1 à 20 mL en perfusion lente ou en bolus.

Par exemple, pour une administration par voie orale, le polymère biocompatible peut être à une concentration comprise de 0,1 à 100 µg/mL en poids de polymère biocompatible par rapport au volume total de la composition, de préférence de 1 à 50 mL, de préférence égale à 5 mL.

Par exemple, pour une administration par voie aérienne, par exemple sous forme de spray par voie nasale, de préférence par inhalation ou nébulisation, le polymère biocompatible peut être à une concentration comprise de 0,1 à 100 µg/mL en poids de polymère biocompatible par rapport au volume total de la composition, de préférence de 1 à 20 mL, de préférence égale à 5 mL. Il peut s'agir par exemple d'une composition, de préférence de 1 à 20 mL, d'une solution aqueuse de OTR4120 à 100 µg/mL ou OTR4131 à 10 µg/mL mise dans un nébuliseur permettant de délivrer de 1 à 3 mL par minutes avec une durée d'administration comprise, par exemple de 5 à 10 minutes.

Dans la présente par peptide on entend un polymère d'acides aminés reliés entre eux par des liaisons peptidiques. L'homme du métier de part ses connaissances générales sait ce qui est entendu par peptide. Il peut s'agir par exemple d'un polymère d'acides aminés reliés entre eux par des liaisons peptidiques comprenant de 2 à 30 acides aminés, par exemple de 2 à 20 acides aminés. Dans la présente par peptide synthétique, on entend tout peptide synthétique connu de l'homme du métier. L'homme du métier de part ses connaissances générales sait ce qui est entendu par peptide synthétique. Il peut s'agir par exemple de peptides obtenus par synthèse chimique par la réaction de condensation du groupe carboxyle d'un acide aminé sur le groupe aminé d'un autre acide aminé, par exemple tel que décrit dans Chan, et al. [18] et/ou Benoiton, 10.1201/9781420027693 [19]. Il peut s'agir par exemple de peptides qui ne sont pas trouvés dans la nature. Il peut s'agir par exemple de peptide synthétisé par la méthode en phase solide (« Solid-Phase Peptide-Synthesis Methodology ») comme décrit dans Fields, 10.1002/0471140864.ps1801s26 [17]).

Dans la présente par protéine toute protéine connue de l'homme du métier. L'homme du métier de par ses connaissances générales sait ce qui est entendu par protéine. Il peut s'agir par exemple de macromolécules biologiques présentes notamment dans toutes les cellules vivantes correspondant à des polymères formés d'une ou de plusieurs chaînes polypeptidiques. La longueur séquence polypeptidiques peut par exemple comprendre de 10 à 30000 acides aminés, par exemple de 10 à 20 000 acides aminés. Chacune des chaînes polypeptidiques est constituée de l'enchaînement de résidus d'acides aminés liés entre eux par des liaisons peptidiques par exemple tel que décrit dans Sanlaville, et al. [20].

Dans la présente par protéine synthétique, on entend toute protéine synthétique connue de l'homme du métier. L'homme du métier de par ses connaissances générales sait ce qui est entendu par protéine synthétique. Il peut s'agir par exemple de protéines obtenues par synthèse chimique, par exemple comprenant des peptides synthétiques et/ou de peptides non synthétiques. Il peut s'agir par exemple de protéines comprenant des peptides synthétiques et/ou de peptides non synthétiques qui ne sont pas présente dans la nature. Il peut s'agir par exemple de protéines obtenues par synthèse chimique dont la structure, les fonctions et les séquences en acides aminés sont identiques à des protéines naturelles.

Dans la présente invention, par vésicule extracellulaire (VE) on entend toute vésicule extracellulaire connue de l'homme du métier. Il peut s'agir de toute vésicule produite par des cellules organismes vivants, par exemple choisi dans le groupe comprenant les *Archae*, *Bacteria*, *Protozoa*, *Chromista*, *Plantae, Fungi* et *Animalia.* Il peut s'agir par exemple de toute vésicule produite et/ou issue par exemple, d'une cellule eucaryote ou procaryote. Il peut s'agir par exemple d'une vésicule produite des cellules eucaryotes ou procaryotes, ladite vésicule comprenant une bicouche lipidique, ne comprenant pas de noyau fonctionnel et ne pouvant se répliquer. Il peut s'agir par exemple d'au moins une vésicule extracellulaire telle que décrite dans le document Raposo, et al., 10.1083/jcb.201211138 [21] et/ou caractérisée dans le document « Les vésicules extracellulaires, Définition, séparation, caractérisation », Wilfrid Boireau* et Céline Elie-Caille**, Med Sci (Paris), Volume 37, Number 12, Décembre 2021, Vésicules extracellulaires, Page(s) 1092 - 1100 ([22], Boireau, et al., 10.1051/medsci/2021201). Il peut s'agir par exemple d'un ou plusieurs des éléments produits par des cellules, notamment en culture *in vitro,* incluant les vésicules ou microvésicules, les exosomes, les corps apoptotiques et les microparticules qui se trouvent dans l'environnement dans lequel les cellules sont maintenues en vie. Les vésicules au sens de la présente invention peuvent comprendre une population hétérogène, dont les éléments sont différenciables notamment en fonction de leur taille/diamètre et de leur contenu.

Dans la présente par exosome on entend une vésicule extracellulaire déversée et/ou excrétée par une cellule dans son environnement dont la taille est comprise de 30 à 120 nm. Il peut s'agir par exemple d'une vésicule extracellulaire déversée et/ou excrétée par une cellule dans son environnement dont la taille est comprise de 30 à 90 nm. Il peut s'agir par exemple d'une vésicule extracellulaire déversée et/ou excrétée par une cellule dans son environnement telle que décrite dans Battistelli, et al., 10.3390/biology9010021 [23]. Il peut s'agir par exemple d'une vésicule extracellulaire déversée et/ou excrétée par une cellule dans son environnement par exemple lors de la fusion des corps multivésiculaires et de la membrane plasmique, par exemple permettant de déverser et/ou excrétée ladite vésicule extracellulaire par une cellule dans son environnement.

Dans la présente par corps apoptotique on entend un type particulier de vésicules extracellulaires (VE) en ce qu'il s'agit de restes de cellules ayant subi une apoptose, ou mort cellulaire programmée. Il peut s'agir par exemple, d'un apoptosome, c'est-à-dire un complexe multiprotéique formé par l'interaction du cytochrome avec l'apaf-1 et la procaspase-9 dans le cytosol, à la suite de la libération du cytochrome depuis la mitochondrie. Il peut s'agir par exemple de vésicules extracellulaires (VE) déversée et/ou excrétée par une cellule mourante. Il peut s'agir par exemple d'un corps apoptotique dont la taille est comprise de 500 nm à 2 µm de diamètre, par exemple de 600 nm à 1µm. Il peut s'agir par exemple d'un corps apoptotique tel que décrit dans Battistelli and Falcieri, 10.3390/biology9010021 [23].

Il peut s'agir d'au moins une vésicule extracellulaire de cellule eucaryote. Dans la présente par vésicule extracellulaire de « cellule eucaryote » on entend toute vésicule extracellulaire de cellule eucaryote connue de l'Homme du métier. Il peut s'agir par exemple d'une vésicule extracellulaire de cellule eucaryote de mammifère ou végétale, par exemple une vésicule extracellulaire de cellule eucaryote animale, humaine ou végétale. Il peut s'agir par exemple d'une vésicule extracellulaire de cellule végétale telle que décrite dans Tan, et al., 10.3389/fphar.2022.1006299 [24]. Il peut s'agir par exemple de toute vésicule extracellulaire de cellule eucaryote de tout tissu biologique de mammifère connue de l'homme du métier. Il peut s'agir par exemple d'une vésicule extracellulaire de cellule eucaryote du tissu conjonctif, du tissu musculaire, du tissu nerveux, du tissu osseux, cartilagineux et/ou du tissu épithéliale. Il peut s'agir par exemple de toute vésicule extracellulaire de cellule eucaryote quel que soit son stade de différenciation, par exemple une vésicule extracellulaire de cellule choisie dans le groupe comprenant les cellules eucaryotes adultes ou embryonnaires, les cellules souches adultes, les cellules pluripotentes natives ou induites (iPS), les cellules multipotentes ou unipotentes, et les cellules différentiées, à l'exception des cellules souches embryonnaires. Il peut s'agir de vésicule extracellulaire de cellules eucaryotes de sang de cordon, de cellules de la moelle osseuse, de cellules du tissu adipeux, de cellules mésenchymateuses, de cellules musculaires, de cellules de tissu, par exemple du tissu osseux articulaire et/ou du tissu musculaire.

Il peut s'agir par exemple d'au moins une vésicule extracellulaire de cellules eucaryotes choisie dans le groupe comprenant des cellules souches adultes, les cellules pluripotentes natives ou induites (iPS pour « induced pluripotent stem cells »), les cellules multipotentes ou unipotentes, et les cellules différentiées ou un quelconque mélange de celles-ci.

Il peut s'agir par exemple de vésicules provenant de cellules choisies parmi des cellules pluripotentes, par exemple les cellules souches embryonnaires ou des cellules somatiques induites à la pluripotence ou cellules souches pluripotentes induites (iPS pour « induced pluripotent cells »), quelque soit l'origine somatique, c'est-à-dire des cellules prélevées chez l'adulte et reprogrammées en cellules pluripotentes par différents procédés incluant, mais non limités à, des adénovirus, des plasmides, des transposons, des virus de Sendaï, des ARNm synthétiques et des protéines recombinantes, par exemple telles que décrites dans le document Takahashi et Yamanaka, « A decade of transcription factor-mediated reprogramming to pluripotency. », Nat Rev Mol Cell Biol. 2016;17:183-93 ([25], Takahashi, et al., 10.1038/nrm.2016.8). Il peut s'agir d'au moins une vésicule extracellulaire de cellules souches embryonnaires lesdites cellules souches embryonnaires n'étant pas issues d'un procédé ayant impliqué la destruction d'un embryon humain.

Il peut s'agir par exemple d'au moins une vésicule extracellulaire de cellules eucaryotes à l'exception des cellules souches embryonnaires.

Il peut s'agir par exemple d'au moins une vésicule extracellulaire de cellules souches adultes, par exemple au moins une vésicule extracellulaire de cellules souches adultes du tissu conjonctif, du tissu musculaire, du tissu nerveux, de tissu adipeux, du tissu osseux, cartilagineux et/ou du tissu épithéliale.

Il peut s'agir par exemple d'au moins une vésicule extracellulaire de cellules eucaryotes adultes. Il peut s'agir par exemple d'au moins une vésicule extracellulaire de cellules eucaryotes embryonnaires. Il peut s'agir par exemple de vésicule extracellulaire de cellules eucaryotes embryonnaires du tissu conjonctif, du tissu musculaire, du tissu nerveux, du tissu adipeux, du tissu placentaire, du tissu osseux, cartilagineux et/ou du tissu épithéliale. Il peut s'agir d'au moins une vésicule extracellulaire de cellules eucaryotes embryonnaires lesdites cellules eucaryotes embryonnaires n'étant pas issues d'un procédé ayant impliqué la destruction d'un embryon humain.

Il peut s'agir par exemple d'au moins une vésicule extracellulaire de cellules pluripotentes ou multipotentes natives. Il peut s'agir par exemple de vésicule extracellulaire de cellules pluripotentes ou multipotentes natives, par exemple les cellules stromales mésenchymateuses, les cellules souche de la moelle osseuse.

Il peut s'agir par exemple d'au moins une vésicule extracellulaire de cellules pluripotentes induites (iPS). Il peut s'agir par exemple de vésicule extracellulaire de cellules pluripotentes induites (iPS) telles que décrites dans Maury Y, Gauthier M, Peschanski M, Martinat C « Les cellules souches pluripotentes humaines - Un outil-clé pour décrypter les mécanismes physiopathologiques » [Human pluripotent stem cells: opening key for pathological modeling]. Med Sci (Paris). 2011 Apr;27(4):443-6. French. ([26], Maury, et al., 10.1051/medsci/2011274023).

Il peut s'agir par exemple d'au moins une vésicule extracellulaire de cellules multipotentes. Il peut s'agir par exemple de vésicule extracellulaire de cellules souches mésenchymateuses, par exemple de cellule souche mésenchymateuse du tissu adipeux, de la moelle osseuse, des tissus de soutien des organes, du tissus osseux, du tissus cartilagineux, du tissu musculaire, de la moelle osseuse. Il peut s'agir par exemple de vésicules provenant de cellules choisies parmi des cellules multipotentes, par exemple des cellules souches mésenchymateuses, ou leurs dérivés différenciés d'origine ectodermique, endodermique et/ou mésodermique, par exemple de cellules choisies parmi des cellules cardiaques, vasculaires, musculaires, rétiniennes, adipocytaires, tendineuses, mésothéliales, fibrillaires, sexuelles, synoviales, cutanées, neurales, médullaires, ostéo-cartilagineuses, hépatiques, rénales, intestinales, hématopoïétiques, et du système immunitaire.

Il peut s'agir par exemple d'au moins une vésicule extracellulaire de cellules unipotentes. Il peut s'agir par exemple de vésicule extracellulaire d'hépatocyte, de kératinocyte, de myoblastes.

Il peut s'agir par exemple d'au moins une vésicule extracellulaire de cellules différentiées. Il peut s'agir par exemple de vésicule extracellulaire d'érythrocytes, de cornéocytes, de myocytes, de pneumocytes primaires.

Il peut s'agir par exemple d'au moins une vésicule extracellulaire comprenant au moins une protéine de liaison aux héparanes sulfates choisie dans le groupe comprenant la chromatine cible de prmt1 :(« chromatin target of prmt1 » (Chtop)), l'inhibiteur de la voie du facteur de tissue (« tissue factor pathway inhibitor » (Tfpi)), la disintégrine et métalloprotéinase avec un motif 4 de thrombospondine de type 1 ( « disintegrin and metalloproteinase with thrombospondin type 1 motif 4 » Adamts4), la protéine angiogénique riche en cystéine 61 (« cysteine-rich angiogenic protein 61 » (Ccn1)) et le facteur neurotrophique dérivé des neurones (« neuron-derived neurotrophic factor » (Ndnf)).II peut s'agir d'au moins une vésicule extracellulaire d'une cellule, ladite vésicules extracellulaire comprenant au moins un peptide et/ou une protéine, protéines d'adhésions cellulaires, des protéines transmembranaires, des protéines sécrétées, des protéines extra-membranaires des protéines intracellulaires, et/ou des lipoprotéines membranaires dont la séquence d'acides aminés comprend un ou plusieurs sites de liaison aux héparanes sulfates. Il peut s'agir d'au moins une vésicule extracellulaire d'une cellule, ladite vésicules comprenant un peptide synthétique et/ou une protéine synthétique comprenant un ou plusieurs sites de liaison aux héparanes sulfates. Il peut s'agir par exemple d'une vésicule extracellulaire d'une cellule comprenant au moins un peptide, une protéine, une protéine d'adhésion cellulaire, une protéine transmembranaire, une protéine sécrétée, une protéine extra-membranaire, une protéine intracellulaire, et/ou une lipoprotéine membranaire avec une séquence peptidique comprenant au moins 3 acides aminés choisi dans le groupe comprenant l'arginine (R), la lysine (L) et l'histidine (H). Il peut s'agir par exemple d'une vésicule extracellulaire d'une cellule comprenant au moins un peptide, une protéine, une protéine d'adhésion cellulaire, une protéine transmembranaire, une protéine sécrétée, une protéine extra-membranaire, une protéine intracellulaire, et/ou une lipoprotéine membranaire avec une séquence peptidique comprenant au moins 3 acides aminés choisi dans le groupe comprenant l'arginine (R), la lysine (L) et l'histidine (H), ledit au moins peptide, protéine, protéine d'adhésion cellulaire, protéine transmembranaire, protéine sécrétée, protéine extra-membranaire, protéine intracellulaire, et/ou lipoprotéine ayant une structure primaire, secondaire, tertiaire et/ou quaternaire comprenant une accumulation et/ou une concentration en acides aminés choisi dans le groupe comprenant l'arginine (R), la lysine (L) et l'histidine (H), ledit au moins peptide, protéine, protéine d'adhésion cellulaire, protéine transmembranaire, protéine sécrétée, protéine extra-membranaire, protéine intracellulaire, et/ou lipoprotéine ayant une charge positive à un pH compris entre 4,8 et 8,0, par exemple entre 7,3 et 7,5.

Il peut s'agir par exemple d'une vésicule extracellulaire d'une cellule comprenant au moins un peptide, une protéine, une protéine d'adhésion cellulaire, une protéine transmembranaire, une protéine sécrétée, une protéine extra-membranaire, une protéine intracellulaire, et/ou une lipoprotéine membranaire comprenant un site de liaison aux héparanes sulfates. Le site de liaison aux héparanes sulfates peut être par exemple tel que décrit dans le document « Importance of spécifie amino acids in protein binding sites for heparin and heparan sulfate » E E Caldwell 1 , V D Nadkarni, J R Fromm, R J Linhardt, J M Weiler PMID: 8729007 DOI: 10.1016/1357-2725(95)00123-9, Int J Biochem Cell Biol. 1996 Feb;28(2):203-16. doi: 10.1016/1357-2725(95)00123-9 ([27], Caldwell, et al., 10.1016/1357-2725(95)00123-9) et/ou tel que décrit par Hileman Ronald E, Fromm Jonathan R, Weiler John M, Linhardt Robert J dans « Glycosaminoglycan-protein interactions: définition of consensus sites in glycosaminoglycan binding proteins» en 1998 dans BioEssays ([28], Hileman, et al., 10.1002/(SICI)1521-1878(199802)20:2<156::AID-BIES8>3.0.CO;2-R). Le site de liaison aux héparanes sulfates peut être par exemple un peptide de séquence BBXaB ou BXaXaBBXaB dans lequel B est n'importe quel acide aminé basique et Xa n'importe quel acide aminé, par exemple tel que décrit dans Wu, H. F., et al. (1995) Blood 85, 421-428 ([29], Wu, et al., 10.1182/blood.V85.2.421.421). Le site de liaison aux héparanes sulfates peut être par exemple un peptide de séquence XaBBBXaXaBBBXaXaBBXa dans lequel B est n'importe quel aminé basique et Xa n'importe quel acide aminé, par exemple tel que décrit dans Andersson, E., et al. (2004) Eur J Biochem 271, 1219-1226 ([30], Andersson, et al., 10.1111/j.1432-1033.2004.04035.x); Sobel, M., et al. (1992) The Journal of biological chemistry 267, 8857-8862 ([31], Sobel, et al., 10.1016/S0021-9258(19)50359-3). Le site de liaison aux héparanes sulfates peut être par exemple un peptide de séquence TXaXaBXaXaTBXaXaXaTBB dans lequel B est n'importe quel aminé basique, Xa est n'importe quel acide aminé et T définit un virage/boucle, par exemple tel que décrit dans Capila, I. et Linhardt, R. J. (2002) Angewandte Chemie International Edition 41, 391-412 ([32], Capila, et al., 10.1002/1521-3773(20020201)41:3<390::AID-ANIE390>3.0.CO;2-B); Hileman, R. E., et al. (1998) BioEssays 20, 156-167 ([28], Hileman, et al., 10.1002/(SICI)1521-1878(199802)20:2<156::AID-BIES8>3.0.CO;2-R); Andersson, E., et al. (2004) Eur J Biochem 271, 1219-1226 ([30], Andersson, et al., 10.1111/j.1432-1033.2004.04035.x).

Il peut s'agir par exemple un peptide, une protéine, une protéine d'adhésion cellulaire, une protéine transmembranaire, une protéine sécrétée, une protéine extra-membranaire, une protéine intracellulaire, une lipoprotéine membranaire comprenant au moins deux, au moins trois acides aminés basiques choisi dans le groupe comprenant l'arginine (R), la lysine (L), l'histidine (H). Il peut s'agir par exemple d'un peptide, une protéine, une protéine d'adhésion cellulaire, une protéine transmembranaire, une protéine sécrétée, une protéine extra-membranaire, une protéine intracellulaire, une lipoprotéine membranaire comprenant une séquence d'acides aminés choisie dans le groupe comprenant XaXbXcXdXeXf, XhXiXjXkXlXmXnXp et XqXrXsXtXuXvXwXyXzX₁X₂ dans lesquelles, Xa, Xd, Xf, Xh, XI, Xm,Xp, Xq, Xr, Xt, Xu, Xw, Xy et Xz sont n'importe quel acide aminé et Xb Xc, Xe, Xi, Xj, Xk,Xn, Xs, Xv, X₁ et X₂ sont n'importe quel acide aminé basique.

Dans la présente, Xa, Xd, Xf, Xh, XI, Xm,Xp, Xq, Xr, Xt, Xu, Xw, Xy et/ou Xz peuvent être indépendamment un acide aminé de la série L ou D.

Il peut s'agir par exemple d'un peptide, une protéine, une protéine d'adhésion cellulaire, une protéine transmembranaire, une protéine sécrétée, une protéine extra-membranaire, une protéine intracellulaire, une lipoprotéine membranaire comprenant une séquence d'acides aminés choisie dans le groupe comprenant RLRARM (SEQ ID NO 28), LRKRLLRD (SEQ ID NO 29), PRRARV (SEQ ID NO 30) et CRLYRK (SEQ ID NO 31).

Dans la présente lorsque le peptide, une protéine, une protéine d'adhésion cellulaire, une protéine transmembranaire, une protéine sécrétée, une protéine extra-membranaire, une protéine intracellulaire, une lipoprotéine membranaire comprenant la séquence d'acides aminés XqXrXsXtXuXvXwXxXyXzX₁X₂, la structure secondaire, tertiaire et/ou quaternaire du peptide, protéine, protéine d'adhésion cellulaire, protéine transmembranaire, protéine sécrétée, protéine extra-membranaire, protéine intracellulaire, lipoprotéine membranaire peut comprendre indépendamment précédemment à l'acide aminé Xq et/ou entre les acides aminés Xu et Xv et/ou entre les acides aminés Xz et X₁, un virage et/ou une boucle T (« turn »).

Il peut s'agir par exemple d'une protéine d'adhésion cellulaire choisie dans le groupe comprenant les collagènes, fibronectines et/ou élastines.

Il peut s'agir par exemple d'une protéine transmembranaire choisie dans le groupe comprenant les tétraspanines par exemple le CD9 et/ou le CD81.

Il peut s'agir par exemple d'une protéine extra-membranaire choisie dans le groupe comprenant les récepteurs membranaires, par exemple choisi dans le groupe comprenant les récepteurs aux facteurs de croissance des fibroblastes (FGFr), les protéines sécrétées, par exemple les facteurs de croissances, les protéines de la cascade de la coagulation et cytokine, par exemple les facteurs de croissance des fibroblastes (FGF), les protéines choisies dans le groupe comprenant le facteur de croissance de l'endothélium vasculaire (VEGF), le facteur de croissance transformant bêta (TGFβ), le facteur neurotrophique issu du cerveau (BDNF), le récepteur des chemokines CXCR/SDF, l'activateur tissulaire du plasminogène (TPA), l'antithrombine, l'antithrombine III, la thrombine, la protéine C réactive, l'inhibiteur de la protéine C, les protéines intracellulaires, par exemple les enzymes superoxydes dismutases ou SOD. Il peut s'agir par exemple d'une lipoprotéine membranaire, par exemple choisie dans le groupe comprenant les apolipoprotéines (APO), Apolipoprotéine E (APOE), les lipoprotéines de différentes densités, par exemple la lipoprotéine de basse densité, les lipoprotéines de très faible densité (VLDL), les lipoprotéines de haute densité (HDL).

Dans la présente par acide aminé acide on entend tout acide aminé acide connu de l'homme du métier. Il peut s'agir par exemple d'un acide aminé acide de la série L ou D. Il peut s'agir par exemple d'un acide aminé acide choisi dans le groupe comprenant l'acide aspartique (D), l'acide glutamique (E), l'asparagine (N), la glutamine (Q), la sérine (S) et la thréonine (T).

Dans la présente par acide aminé basique on entend tout acide aminé basique connu de l'homme du métier. Il peut s'agir par exemple d'un acide aminé basique de la série L ou D. Il peut s'agir par exemple d'un acide aminé basique choisi dans le groupe comprenant l'arginine (R), la lysine (K), et/ou l'histidine (H).

Avantageusement, les acides aminés Xa, Xd, Xf, Xh, XI, Xm et Xp peuvent être indépendamment, par exemple lorsque le peptide et/ou protéine est sous forme tertiaire ou quaternaire, orienté vers l'intérieur et/ou l'extérieur dudit peptide et/ou de la protéine.

Il peut s'agir par exemple d'une vésicule extracellulaire comprenant un peptide et/ou une protéine et/ou un peptide de synthèse et/ou une protéine de synthèse comme par exemple protéines d'adhésions cellulaires, des protéines transmembranaires, des protéines extra-membranaires, des protéines sécrétées, des protéines intracellulaires et/ou des lipoprotéines membranaires et comprenant un site de liaison aux héparanes sulfates tel que décrit dans le document Heparin-Binding Domains in Vascular Biology Eva M. Muñoz and Robert J. Linhardt, Arterioscler Thromb Vase Biol. 2004 Sep; 24(9): 1549-1557. doi: 10.1161/01.ATV.0000137189.22999.3f ([33], Munoz, et al., 10.1161/01.ATV.0000137189.22999.3f). Il peut s'agir par exemple d'au moins une vésicule extracellulaire comprenant au moins une protéine choisie dans le groupe comprenant la protéine cible chromatine de prmt1 (« Chromatin target of prmt1 ») CHTOP (UniProt : Q9Y3Y2), la protéine inhibiteur de la voie du facteur tissulaire TFPI (UniProt : P10646), la protéine désintégrine et métalloprotéinase avec des motifs de thrombospondine 4 (« Disintegrin And Metalloproteinase with Thrombospondin Motifs 4 », ADAMTS4, UniProt :O75173-ATS4), la protéine cysteine-rich angiogenic protein 61 également désignée Ccn1 (CCN1, UniProt: O00622), le facteur neurotrophique dérivé des neurones humains (« Human Neuron Derived Neurotrophic Factor » Ndnf, UniProt :Q8TB73), la fibronectine (UNiProt : P02751-FINC) ou le collagène 1 (UNiProt : P02452- CO1A1), ladite au moins une protéine comprenant au moins un site de liaison aux héparanes sulfates. Dans la présente pour l'identification des protéines, la nomenclature UniProt Consortium est utilisée UniProt: the Universal Protein Knowledgebase in 2023 ([34], UniProt, 10.1093/nar/gkac1052).

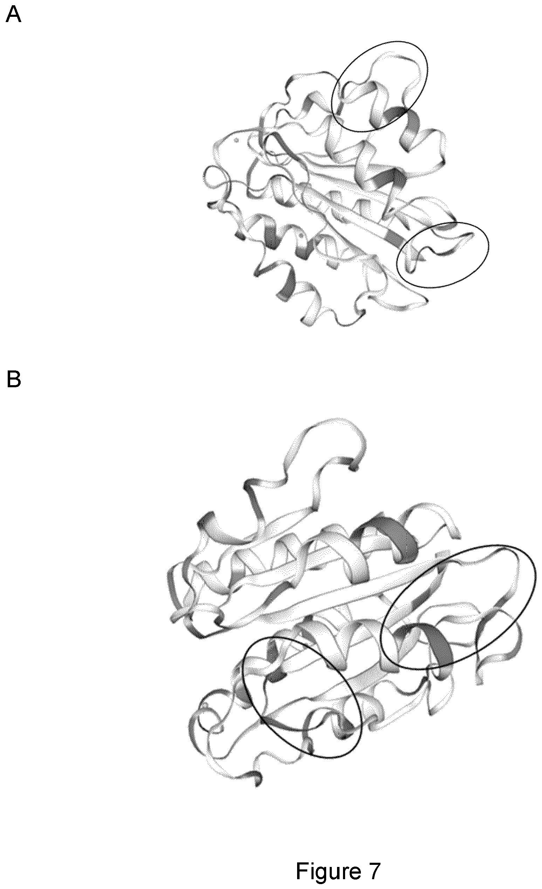

Avantageusement, les acides aminés basique peuvent être chargés positivement à pH physiologique due à la présence d'un ou de plusieurs acides aminés basiques, à savoir un pH compris entre 7 et 7,7, de préférence 7,4 et peuvent avantageusement former un groupement/une zone chargé/e positivement au sein de la protéine ou du peptide. Avantageusement, lesdits acides aminés basiques peuvent former un groupement/une zone chargé/e positivement au sein de la protéine ou du peptide, par exemple lorsqu'il(elle) est sous forme quaternaire. Il peut s'agir par exemple d'une protéine ou d'un peptide comprenant un groupement/une zone chargé/e positivement au sein de la protéine ou du peptide par exemple identique ou similaire au collagène 7 (UniProt : CO7A1-Q02388). Il peut s'agir par exemple d'une protéine ou du peptide comprenant une séquence peptidique comprenant au moins un, au moins deux, au moins trois, au moins quatre, au moins cinq, au moins six, au moins sept, au moins huit peptide(s) choisi(s) dans le groupe comprenant RVRAQHRERVT (SEQ ID NO 1) : SVRLRGLRPL (SEQ ID NO 2), ARGYRLEWRR (SEQ ID NO 3), HRAEATRRVLER (SEQ ID NO 4), PGRRQH (SEQ ID NO 5), RRRGPKG (SEQ ID NO 6), RGERGEK (SEQ ID NO 7), RGLKGERGVK (SEQ ID NO 8), RWYHR (SEQ ID NO 9). Il peut s'agir par exemple d'une protéine avec un structure tertiaire tel que représenté sur la figure 7.

Il peut s'agir par exemple d'au moins une vésicule extracellulaire comprenant au moins une protéine choisie dans le groupe comprenant des protéines transmembranaires par exemple des tétraspanines, par exemple CD9, CD63, CD81 et/ou CD82.

Il peut s'agir par exemple d'au moins une vésicule extracellulaire comprenant au moins une protéine choisie dans le groupe comprenant des protéines cytosoliques, par exemple Alix et/ou TSG101.

Il peut s'agir par exemple d'au moins une vésicule extracellulaire comprenant au moins une protéine choisie dans le groupe comprenant des annexines.

Il peut s'agir par exemple d'au moins une vésicule extracellulaire comprenant au moins une protéine choisie dans le groupe comprenant des immuno-régulateur (MHC).

Il peut s'agir par exemple d'au moins une vésicule extracellulaire comprenant au moins une protéine choisie dans le groupe comprenant des intégrines, par exemple D2C9

Il peut s'agir par exemple d'au moins une vésicule extracellulaire comprenant au moins une protéine choisie dans le groupe comprenant des immunoglobulines, par exemple CD90.

Il peut s'agir par exemple d'au moins une vésicule extracellulaire comprenant au moins une protéine choisie dans le groupe comprenant CD73 et CD44.

Il peut s'agir par exemple d'au moins une vésicule extracellulaire extraite et/ou isolée à partir d'un échantillon d'un liquide et/ou fluide biologique. Il peut s'agir par exemple d'au moins une vésicule extracellulaire extraite et/ou isolée à partir d'un échantillon d'un liquide biologique choisi dans le groupe comprenant sang, urine, salive, sperme, liquide céphalo-rachidien, sérum, liquide lacrymal, liquide amniotique, liquide synovial, lait,

Il peut s'agir d'une vésicule extracellulaire de cellule procaryote. Dans la présente par « vésicule extracellulaire de cellule procaryote» on entend toute vésicule extracellulaire de cellule procaryote connue de l'Homme du métier. Il peut s'agir par exemple de vésicules extracellulaires de bactérie.

Le choix des cellules, et par voie de conséquence des vésicules qui sont utilisées pour la mise en oeuvre de la présente invention, est bien sûr dépendant de la cible thérapeutique visée lors de l'utilisation de la composition de la présente invention, par exemple de la lésion tissulaire et/ou du tissu déficient à réparer.

Selon l'invention, la préparation de vésicules utilisables pour la mise en oeuvre de la présente invention peut être réalisée à partir de tout procédé adapté connu de l'homme du métier. Il peut s'agir par exemple d'un procédé *in-vitro* comprenant la culture desdites cellules, les vésicules étant sécrétées par les cellules en question dans leur milieu de culture dont on a préalablement vérifié qu'il ne contient pas (ou très peu) de vésicules qui représenteraient naturellement un facteur confondant. Les vésicules extracellulaires sont prélevées de ces milieux conditionnés. Les documents suivants décrivent des procédés de culture de cellules et la préparation de vésicules issues de ces cultures utilisables pour la mise en oeuvre de la présente invention, à partir de différents types cellulaires :
- Akbar, et al., 10.3390/cells11020186 [35],
- Clos-Sansalvador, et al., 10.1016/j.ejcb.2022.151227 [36]
- Liangsupree, et al., 10.1016/j.chroma.2020.461773 [37],
- Brennan, et al., 10.1038/s41598-020-57497-7 [38].

Il peut s'agir d'une vésicule extracellulaire comprenant en outre au moins un autre ingrédient actif ; particulièrement un autre ingrédient thérapeutiquement actif ; ledit ingrédient actif étant internalisé et/ou adsorbé et/ou greffé sur les vésicules extracellulaires.

L'ingrédient actif peut être par exemple tout produit pharmaceutiques connus de l'homme du métier. Il peut s'agir par exemple d'un ingrédient actif utilisé par exemple dans le traitement de maladies opportunes, par exemple provoquée par un microorganisme, exemple par un virus ou une bactérie, qui peuvent se développer chez un patient présentant une lésion tissulaire. L'ingrédient actif peut être par exemple au moins un antibiotique, au moins un anti-inflammatoire, au moins un antiviral. Il peut s'agir par exemple de vésicule extracellulaire comprenant en outre au moins un autre ingrédient actif ; particulièrement un autre ingrédient thérapeutiquement actif ; telle que décrite dans Panigrahi, et al., 10.1016/j.tranon.2022.101439 [39].

Dans la présente, par « composition pharmaceutique » on entend toute forme de composition pharmaceutique connue de l'Homme du métier. Dans la présente, la composition pharmaceutique peut être par exemple une solution injectable. Il peut s'agir par exemple une solution injectable, par exemple pour une injection locale ou systémique, par exemple en sérum physiologique, en solution glucose injectable, en présence d'excipients, par exemple de Dextrans, par exemple a des concentrations connues de l'homme du métier, par exemple du microgramme à quelques milligrammes par mL. La composition pharmaceutique peut être par exemple un médicament destiné à une administration orale choisie dans le groupe comprenant une formulation liquide, une forme posologique effervescente orale, une poudre orale, un système multiparticule, une forme galénique orodispersible.

Par exemple, lorsque la composition pharmaceutique est pour administration orale, elle peut être sous la forme d'une formulation liquide choisie dans le groupe comprenant une solution, un sirop, une suspension, une émulsion. Lorsque la composition pharmaceutique est sous la forme d'une forme posologique effervescente orale, elle peut être sous une forme choisie dans le groupe comprenant les comprimés, les granules, les poudres. Lorsque la composition pharmaceutique est sous la forme d'une poudre orale ou un système multiparticulaire, il peut être sous une forme choisie dans le groupe comprenant des billes, des granulés, des mini-comprimés et les microgranules. Lorsque la composition pharmaceutique est sous la forme d'une forme posologique orodispersible, elle peut être sous une forme choisie dans le groupe comprenant des comprimés orodispersibles, gaufrettes lyophilisées, films minces, un comprimé à mâcher, d'un comprimé, d'une capsule ou d'une gomme médicale à mâcher.

Selon la présente invention, la composition pharmaceutique peut être une composition pharmaceutique pour administration par voir orale, par exemple buccale et/ou sublingual, par exemple choisie dans le groupe comprenant les comprimés buccaux ou sublinguaux, les pastilles, gouttes, une solution pour pulvérisations.

Selon la présente invention, la composition pharmaceutique peut être une composition pharmaceutique pour administration topique, transdermique, par exemple choisie dans le groupe comprenant les pommades, crèmes, gels, lotions, patchs et mousses.

Selon la présente invention, la composition pharmaceutique peut être une composition pharmaceutique pour administration par voie respiratoire ou nasale, par exemple sous la forme d'un aérosol, par exemple administré avec un nébuliseur et/ou inhalateur.

Selon la présente invention, la composition peut être une composition pour administration nasale ou respiratoire nasale ou respiratoire, par exemple choisie dans le groupe comprenant des gouttes nasales, spray nasal, de la poudre nasale, des aérosols, par exemple des aérosols et/ou spray nasal à gaz comprimé, ou des nébuliseurs

Selon la présente invention, la composition pharmaceutique peut être une composition pharmaceutique adaptée pour administration intra-pulmonaire.

Selon la présente invention, la composition pharmaceutique peut être une composition pharmaceutique pour administration parentérale, par exemple sous-cutanée, intramusculaire, intraveineuse, intra-artérielle, intracrânienne, intrathécale.

Selon la présente invention, la composition pharmaceutique peut être une composition pharmaceutique pour administration par voie oculaire, par exemple choisie dans le groupe comprenant un collyre, une pommade ophtalmique, un gel ophtalmique, un bain oculaire.

La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée. Cet autre ingrédient peut être par exemple un ingrédient actif utilisé par exemple dans le traitement de maladies opportunes qui peuvent se développer chez un patient présentant une lésion tissulaire provoquée par un microorganisme, par exemple par un virus ou une bactérie. Il peut s'agir également de produits pharmaceutiques connus de l'homme du métier, par exemple des antibiotiques, anti-inflammatoires, des antiviraux, des anticancéreux.

La composition de la présente invention peut également comprendre au moins un autre ingrédient actif, particulièrement un autre ingrédient thérapeutiquement actif, par exemple pour une utilisation simultanée, séparée ou étalée dans le temps suivant la formulation galénique utilisée. Cet autre ingrédient peut être par exemple un ingrédient actif utilisé par exemple dans le traitement de maladies opportunes ou une vitamine, par exemple la vitamine C utilisée à haute dose, par exemple 50 à 100 mg/kg /jour ou un antalgique ou un antibiotique ou un bronchodilatateur par exemple le salbutamol, ou un corticoïde par exemple le Methylprednisolone ou un antiviral, par exemple l'interféron alfa-2b ou le lopinavir.

Dans la présente, l'administration du polymère biocompatible et de l'acide hyaluronique peut être simultanée, successive ou concomitante.

Selon l'invention, au moins une des administrations peut être réalisée par voie topique, orale, respiratoire ou par injection, de préférence par voie respiratoire. Les deux administrations peuvent être réalisées de la même manière ou différemment. Par exemple, l'administration du polymère biocompatible et de l'acide hyaluronique peut être faite par voie respiratoire. L'administration peut être également fonction de la zone et/ou du tissu biologique à traiter.

Selon l'invention, la composition peut être, par exemple, administrée une seule fois.

Selon l'invention, la composition peut être en outre, par exemple, administrée quotidiennement, bi quotidiennement et hebdomadairement ou moins. Il peut s'agir par exemple d'une administration une fois par jour, deux fois par jour ou moins, par exemple une fois tous les deux jours, ou par semaine.

Selon l'invention, et le mode d'administration la composition peut être, par exemple, pour une composition saline administrée quotidiennement, bi quotidiennement et hebdomadairement ou moins. Il peut s'agir par exemple d'une administration une fois par jour, deux fois par jour ou moins.

Selon l'invention, la composition peut être, par exemple, administrée sur une durée de 1 jour à 3 mois, par exemple pendant 2 mois, par exemple pendant 1 mois, par exemple pendant une semaine. Par exemple, la composition peut être administrée sur une période de 1 à 3 semaines, par exemple avec une fréquence d'administration tous les jours ou tous les deux jours.

Par exemple, lorsque la composition est sous une forme adaptée à une administration par voie respiratoire, la composition peut être administrée de préférence avec une fréquence d'administration tous les deux ou trois jours.

Selon l'invention, la composition peut être, par exemple, administrée quotidiennement, bi quotidiennement et hebdomadairement. Il peut s'agir par exemple d'une administration une fois par jour, deux fois par jour ou plus.

Selon l'invention, la composition peut être, par exemple, administrée sur une durée de 1 jour à 3 mois, par exemple pendant 2 mois. Par exemple, la composition peut être administrée sur une période de 3 mois avec une fréquence d'administration tous les jours.

La présente invention a également pour objet une composition pharmaceutique comprenant un biopolymère de formule AaXxYy ou AaXxYyZz et au moins une vésicule extracellulaire comme médicament.

Selon l'invention, la vésicule extracellulaire est telle que définie ci-dessus.

Selon l'invention, le biopolymère de formule AaXxYy ou AaXxYyZz est tel que défini ci-dessus.

Selon l'invention, la composition pharmaceutique est telle que définie ci-dessus.

Selon l'invention la fréquence d'administration de la composition pharmaceutique peut être telle que définie ci-dessus.

Selon l'invention, le mode et/ou la voie d'administration la composition pharmaceutique peut être tel(s) que défini(s) ci-dessus.

La présente invention a également pour objet la composition pharmaceutique comprenant un biopolymère de formule AaXxYy ou AaXxYyZz et au moins une vésicule extracellulaire pour son utilisation pour la prévention et/ou le traitement de lésions tissulaires.

Selon l'invention, la vésicule extracellulaire est telle que définie ci-dessus.

Selon l'invention, le biopolymère de formule AaXxYy ou AaXxYyZz est tel que défini ci-dessus.

Selon l'invention, la composition pharmaceutique est telle que définie ci-dessus.

Selon l'invention la fréquence d'administration de la composition pharmaceutique peut être telle que définie ci-dessus.

Selon l'invention, le mode et/ou la voie d'administration la composition pharmaceutique peut être tel(s) que défini(s) ci-dessus.

Dans la présente par « lésions tissulaires » on entend toute lésion de tout tissu biologique d'un mammifère connu de l'homme du métier. Il peut s'agir par exemple d'un dommage ou un changement anormal dans le tissu d'un organisme, par exemple due à une blessure ou une maladie. Il peut s'agir de toute lésion tissulaire connue de l'homme du métier quelque soit la forme et/ou taille de la lésion. Il peut s'agir par exemple d'une lésion tissulaire comprenant une inflammation ou non, de préférence une lésion tissulaire sans inflammation. Il peut s'agir par exemple d'une lésion telle que décrit dans Adolphs, 10.1016/j.neuron.2016.05.014 [43]. Il peut s'agir par exemple d'une lésion du tissu conjonctif, du tissu musculaire, du tissu nerveux, du tissu osseux, cartilagineux et/ou du tissu épithéliale. Il peut s'agir par exemple de toute lésion de tout organe, organelle de mammifère connu de l'homme du métier. Il peut s'agir par exemple d'une lésion de la peau, des muqueuses, des tissus du tractus digestif, de tissus du tractus gastro-intestinal, du système digestif alimentation et excrétion, du tractus génital, du système reproducteur, du système optique, olfactif ou auditif, du système sensoriel, du système circulatoire et/ou cardiovasculaire, du système respiratoire, du système musculaire, du système locomoteur, du système nerveux central, du système nerveux périphérique. Il peut s'agir par exemple d'une lésion du tissu gastrique, d'une lésion buccale, par exemple une lésion parodontale, une lésion buccale inflammatoires, par exemple une mucite, d'une lésion de la cornée, d'une lésion tympanique, d'une lésion de la cochlée, d'une lésion de la peau, par exemple une plaie, une plaie chronique, par exemple une plaie du diabétique, une plaie ulcéreuse, un escarre, une brulure cutanée, une plaie nécrosante, une lésion veineuse, une lésion ischémique, par exemple une nécrose ischémique, une lésion due à un infarctus, par exemple un infarctus du myocarde, une lésion osseuse, par exemple une fracture, une fracture avec défaut osseux, une ostéonécrose (« non union bone fracture »), une lésion ostéochondrale, une lésion cartilagineuse, une lésion tendineuse, une lésion chirurgicale, une lésion due à une opération chirurgicale, une lésion due à un traitement médicale, par exemple une radiothérapie, une lésion du tissu nerveux, par exemple une lésion du cerveau, par exemple une lésion due à une exérèse d'une tumeur, une lésion de la moelle épinière, une lésion de fibre nerveuse, par exemple du système locomoteur et/ou sensoriel, par exemple une lésion neuromusculaire, une lésion du système respiratoire, par exemple des lésions pulmonaires, une lésion du système circulatoire, par exemple une lésion des artères et/ou des vaisseaux, une lésion du système digestif, une lésion rénal, une lésion hépatique, urinaire, tissus de la sphère orale.

Il peut s'agir également de toute lésion de tout tissu biologique d'une plante connue de l'homme du métier. Il peut s'agir par exemple de toute lésion de tout organe, organelle de plante connu de l'homme du métier.

Dans la présente, une "quantité efficace" est une quantité qui permet de traiter une lésion, ou de produire un effet mentionné. Par exemple, une quantité efficace peut être une quantité efficace pour réduire la progression ou la gravité de l'état ou des lésions traités. La détermination d'une quantité thérapeutiquement efficace est largement à la portée de l'homme du métier. Le terme "quantité efficace" est destiné à inclure une quantité d'un composé décrit ici, ou une quantité d'une combinaison de composés décrits ici, par exemple, qui est efficace pour traiter ou prévenir une lésion, ou pour traiter les symptômes de la lésion, chez un hôte. Par conséquent, une "quantité efficace" signifie généralement une quantité qui produit l'effet désiré.

Les termes "traiter", "traiter" et "traitement" comprennent (i) la prévention de l'apparition d'une maladie, d'un état pathologique ou médical (par exemple, la prophylaxie) ; (ii) l'inhibition de la lésion ou l'arrêt de son développement ; (iii) le soulagement de la lésion ; et/ou (iv) la diminution des symptômes associés à la lésion. Ainsi, les termes "traiter", "traitement" et "soigner" s'étendent à la prophylaxie et incluent la prévention, l'empêchement, la diminution, l'arrêt ou l'inversion de la progression ou de la gravité de l'état ou des symptômes traités, par exemple de la lésion traitée. Ainsi, le terme "traitement" comprend l'administration médicale, thérapeutique et/ou prophylactique, selon le cas.

La présente invention se rapporte également à un procédé de traitement d'un patient ayant subi lésion tissulaire comprenant l'administration d'une composition pharmaceutique comprenant un biopolymère de formule AaXxYy ou AaXxYyZz et au moins une vésicule extracellulaire.

Selon l'invention, la vésicule extracellulaire est telle que définie ci-dessus.

Selon l'invention, le biopolymère de formule AaXxYy ou AaXxYyZz est tel que défini ci-dessus.

Selon l'invention, la composition pharmaceutique est telle que définie ci-dessus.

Selon l'invention la fréquence d'administration de la composition pharmaceutique peut être telle que définie ci-dessus.

Selon l'invention, le mode et/ou la voie d'administration la composition pharmaceutique peut être tel(s) que défini(s) ci-dessus.

Selon l'invention, le patient peut être tout mammifère. Il peut s'agir par exemple d'un animal ou d'un être humain.

La présente invention a également pour objet un kit pharmaceutique pour la prévention et/ou le traitement de lésions tissulaires comprenant :
i. un polymère biocompatible, et
ii. au moins une vésicule extracellulaire.

Le polymère biocompatible est tel que défini ci-dessus.

La vésicule extracellulaire est telle que définie ci-dessus.

Les inventeurs ont démontré de manière surprenante et inattendue que les vésicules extracellulaires expriment des protéines permettant avantageusement, lorsque qu'elles sont associées au polymère biocompatible selon l'invention, une fixation dudit polymère auxdites vésicules extracellulaires avec une forte affinité. En outre, les inventeurs ont démontré de manière surprenante et inattendue que les polymères biocompatibles selon l'invention peuvent se fixer, par exemple par des liaisons intermoléculaires attractives, par exemple résultant de l'interaction dipôle-dipôle, par exemple via des liaisons hydrogènes, des liaisons hydrophobes, des liaisons ou forces de van der Waals, de préférence des liaisons électrostatiques et/ou par des liaisons ioniques. En particulier, les inventeurs ont démontré de manière surprenante que les polymères biocompatibles selon l'invention peuvent se fixer sur des protéines présentes à la surface membranaire desdites vésicules extracellulaires, par exemple des peptides et/ou protéines mentionnées précédemment, par exemple des protéines extracellulaires et/ou des protéines transmembranaires par exemple la protéine Chtop (« Chromatin target of prmt1 »), la protéine inhibiteur de la voie du facteur tissulaire (Tfpi), la protéine désintégrine et métalloprotéinase avec des motifs de thrombospondine 4 (« Disintegrin And Metalloproteinase with Thrombospondin Motifs 4 »(Adamts4)), la protéine cysteine-rich angiogenic protein 61 également désignée Ccn1 et/ou le Facteur neurotrophique dérivé des neurones humains (« Human Neuron Derived Neurotrophic Factor » (Ndnf))

Les inventeurs ont démontré de manière surprenante et inattendue que la combinaison des vésicules extracellulaires et d'un polymère biocompatible selon l'invention permet avantageusement de protéger des protéines, par exemple les protéines chromatine cible de prmt1 (« chromatin target of prmt1 » (Chtop)), l'inhibiteur de la voie du facteur de tissue (« tissue factor pathway inhibitor » (Tfpi)), la disintegrine et métalloprotéinase avec un motif 4 de thrombospondine de type 1 ( « disintegrin and metalloproteinase with thrombospondin type 1 motif 4 » Adamts4), la protéine angiogénique riche en cystéine 61 (« cysteine-rich angiogenic protein 61 » (Ccn1)) et le facteur neurotrophique dérivé des neurones (« neuron-derived neurotrophic factor » (Ndnf)) présentent à la surface desdites vésicules extracellulaires. En particulier, les inventeurs ont démontré de manière surprenante et inattendue que la combinaison de vésicules extracellulaires et d'un polymère biocompatible selon l'invention permet avantageusement de protéger des protéines présentent à la surface desdites vésicules extracellulaires de la protéolyse.

Les inventeurs ont également démontré de manière surprenante et inattendue que le polymère biocompatible selon l'invention permet avantageusement de protéger des protéines présentent à la surface desdites vésicules extracellulaires de la protéolyse par exemple via une fixation dudit polymère biocompatible auxdites protéines.

Les inventeurs ont démontré de manière surprenante que la combinaison selon l'invention permet avantageusement une fixation du polymère biocompatible selon l'invention à la surface de la vésicule extracellulaire et permet en outre et de manière surprenante de protéger ladite vésicule de la protéolyse.

La présente invention a également pour objet une vésicule extracellulaire comprenant à sa surface au moins un polymère biocompatible de formule générale (I) ou (II).

Le polymère biocompatible de formule (I) ou (II) est tel que défini ci-dessus.

La vésicule extracellulaire est telle que définie ci-dessus.

Dans la présente par à la surface de vésicule extracellulaire on entend la surface de la membrane externe de la vésicule. Il peut s'agir par exemple de la surface de la membrane externe de la vésicule faisant face au milieu extérieur. Il peut s'agir par exemple d'une couche coronale exo-faciale qui entoure la membrane de la vésicule extracellulaire. La membrane de la vésicule faisant face au milieu extérieur et/ou couche coronale exo-faciale peut comprendre des molécules, par exemple des peptides et/ou protéines dérivées des cellules dont elles sont issues et acquises par le biais d'interactions électrostatiques avec des molécules extracellulaires.

Avantageusement, les peptides et/ou protéines présentent à la surface des vésicules extracellulaires peuvent contribuer aux propriétés moléculaires, par exemple à l'adressage des vésicules extracellulaires, par exemple sur des cellules, par exemple via des récepteurs spécifiques et/ou des protéines et/ou des peptides. Avantageusement les vésicules adressées sur lesdites cellules peuvent être internalisées dans lesdites cellules. Avantageusement, les peptides et/ou protéines présentent à la surface des vésicules extracellulaires peuvent contribuer aux propriétés moléculaires physiques, par exemple les modifications de la fluidité membranaire, et/ou électrostatiques, par exemple en donnant à la vésicule extracellulaire une charge globale spécifique, desdites vésicules extracellulaires. Cette couche coronale régule les interactions intercellulaires, la mobilité et la biodistribution des vésicules extracellulaires, et contribue souvent à de multiples caractéristiques clés pour l'identification, la classification et l'isolement par affinité des vésicules extracellulaires ([13], Hallal, et al., 10.1002/jev2.12260) ([14], Buzas, et al., 10.1007/s00281-018-0682-0).

Avantageusement, le polymère biocompatible de formule (I) ou (II) peut être adhéré, absorbé et/ou fixé à la surface de vésicule extracellulaire. Par exemple, la constante d'affinité (KD) entre la vésicule extracellulaire et le polymère biocompatible de formule (I) ou (II) peut être comprise de 10⁻¹² à 10⁻⁸ M, par exemple de 10⁻¹¹ à 10⁻⁸ M.

Avantageusement, la vésicule extracellulaire comprenant à sa surface au moins un polymère biocompatible de formule générale (I) ou (II) peut être adressée aux sites de lésion tissulaires, par exemple comprenant des peptides et/ou des protéines possédant des sites de liaison, par exemple au héparane sulfate tel que mentionné ci-dessus.

La présente invention a également pour objet une vésicule extracellulaire comprenant à sa surface au moins un polymère biocompatible de formule générale (I) ou (II) pour son utilisation dans la prévention et/ou dans le traitement d'une lésion tissulaire.

La lésion tissulaire est telle que définie ci-dessus.

La présente invention a également pour objet un procédé de préparation de vésicules extracellulaires avec au moins un polymère biocompatible comprenant les étapes de :
a. culture d'au moins une cellule dans un milieu M1
b. culture de ladite au moins une cellule obtenue à l'étape a dans un milieu M2 comprenant un polymère biocompatible,
c. Récupération des vésicules extracellulaires obtenues à l'étape b. à partir du milieu M2.

Le polymère biocompatible est tel que défini ci-dessus.

La vésicule extracellulaire est telle que définie ci-dessus.

Dans la présente, la cellule est telle que définie ci-dessus. Il peut s'agir par exemple d'une cellule eucaryote de mammifère ou végétale. Il peut s'agir par exemple d'une cellule eucaryote de tout tissu biologique de mammifère connue de l'homme du métier. Il peut s'agir par exemple d'une cellule eucaryote du tissu conjonctif, du tissu musculaire, du tissu nerveux, du tissu osseux, cartilagineux et/ou du tissu épithéliale. Il peut s'agir par exemple de toute cellule eucaryote quelle que soit son stade de différenciation, par exemple une cellule choisie dans le groupe comprenant les cellules eucaryotes adultes ou embryonnaires, les cellules souches adultes, les cellules pluripotentes natives ou induites (iPS), les cellules multipotentes ou unipotentes, et les cellules différentiées, à l'exception des cellules souches embryonnaires. Il peut s'agir de cellule eucaryote de sang de cordon, de cellules de la moelle osseuse, de cellules du tissu adipeux, de cellules mésenchymateuses, de cellules musculaires, de cellules de tissu, par exemple du tissu osseux articulaire et/ou du tissu musculaire. Il peut s'agir par exemple d'une cellule eucaryote choisie dans le groupe comprenant des cellules souches adultes, les cellules pluripotentes natives ou induites (iPS), les cellules multipotentes ou unipotentes, et les cellules différentiées ou un quelconque mélange de celles-ci. Il peut s'agir par exemple d'une cellule souche adulte, par exemple une cellule souche adulte du tissu conjonctif, du tissu musculaire, du tissu nerveux, du tissu osseux, cartilagineux et/ou du tissu épithéliale. Il peut s'agir par exemple d'une cellule eucaryote adulte. Il peut s'agir par exemple d'une cellule eucaryote embryonnaires.

Il peut s'agir par exemple d'une cellule eucaryote embryonnaire du tissu conjonctif, du tissu musculaire, du tissu nerveux, du tissu osseux, cartilagineux et/ou du tissu épithéliale Il peut s'agir par exemple d'une cellule pluripotente natives Il peut s'agir par exemple de cellules Il peut s'agir par exemple d'une cellule pluripotente induite (iPS). Il peut s'agir par exemple d'une pluripotente induite (iPS) telle que décrite dans Maury Y, Gauthier M, Peschanski M, Martinat C [Ref 10.1051/medsci/2011274023]. Les cellules souches pluripotentes humaines - Un outil-clé pour décrypter les mécanismes physiopathologiques [Human pluripotent stem cells: opening key for pathological modeling]. Med Sci (Paris). 2011 Apr;27(4):443-6. French. doi: 10.1051/medsci/2011274023. Epub 2011 Apr28. PMID: 21524412 [27] Il peut s'agir par exemple d'une cellule multipotente. Il peut s'agir par exemple d'une cellule souche mésenchymateuse, par exemple de cellule souche mésenchymateuse du tissu adipeux, de la moelle osseuse, des tissus de soutien des organes, du tissus osseux, du tissus cartilagineux, du tissu musculaire, de la moelle osseuse. Il peut s'agir par exemple d'une cellule unipotente. Il peut s'agir par exemple d'hépatocyte, de kératinocyte, de myoblaste. Il peut s'agir par exemple d'une cellule différentiée. Il peut s'agir par exemple d'érythrocytes, de cornéocytes, de myocytes, de chondrocytes, d'hépatocytes, de cholangiocytes, synoviocytes, ovocytes, spermatozoïdes

Il peut s'agir d'une cellule procaryote. Il peut s'agir par exemple de toute cellule procaryote connue de l'homme du métier susceptible de produire au moins une vésicule extracellulaire. Il peut s'agir par exemple de cellule bactérienne.

Dans la présente par milieu M1, on entend tout milieu de culture de connu de l'homme du métier adapté à la culture de cellules eucaryotes, procaryote ou végétale. Il peut d'agir par exemple d'un milieu de culture de cellules eucaryotes disponible dans le commerce. Il peut s'agir par exemple d'un milieu de culture de cellules eucaryotes choisi dans le groupe comprenant le milieu Dulbecco's Modified Eagle's Medium (DMEM) - low glucose (D4655, Sigma-Aldrich), supplémenté de 1 % de pénicilline-streptomycine (P4333, Sigma-Aldrich), de 1 % de L-glutamine (G7513, Sigma-Aldrich) et de 10 % de sérum de veau foetal (FB-1001, Biosera). Il peut d'agir par exemple d'un milieu de culture de cellules procaryotes disponible dans le commerce. Il peut s'agir par exemple d'un milieu de culture de cellules procaryotes, disponible dans le commerce par exemple le milieu de lysogénie ou lysogeny broth abrégé LB, par exemple le milieu LB, L2542, commercialisé par la société Sigma-Aldrich. Il peut s'agir par exemple d'un milieu de culture de cellules végétales disponible dans le commerce comme par exemple le milieu B5 de Gamborg (G5893, Sigma-Aldrich).

L'homme du métier, de part ces connaissances générales, saura adapter le milieu de culture en fonction des cellules utilisées.

Dans la présente par milieu M2, on entend tout milieu connu de l'homme du métier adapté à l'incubation de cellules et l'isolement de vésicules extracellulaire. Il peut s'agir par exemple, pour des cellules eucaryote, du milieu Dulbecco's Modified Eagle's Medium (DMEM) - low glucose (D4655, Sigma-Aldrich), supplémenté de 1 % de pénicilline-streptomycine (P4333, Sigma-Aldrich) et de 2 mM de L-glutamine (G7513, Sigma-Aldrich) i

Selon l'invention, le milieu M2 peut comprendre une concentration polymère biocompatible de 1 picogrammes/mL à 10 mg/mL, de préférence de 0,1 à 100 µg/mL

Dans la présente, la durée de l'étape a) de culture peut être comprise de 3 à 5 jours.

Dans la présente, la culture de cellules de l'étape a) peut être réalisée sous une atmosphère contrôlée comprenant de 5 % de CO₂.

Selon l'invention, l'étape a) de culture de cellules peut être réalisée à une température comprise entre 4 °C et 45 °C, par exemple à 37°C ou 4°C. Par exemple lorsque les cellules sont des cellules eucaryotes, par exemple de mammifères, la culture de cellules peut être réalisée à une température de 37°C. Par exemple lorsque les cellules sont des cellules procaryotes par exemple des bactéries, des champignons, les levures et les cellules végétales la température, la culture de cellules peut être réalisée à une température de 4 °C. Par exemple lorsque les cellules sont des bactéries thermorésistantes, par exemple Escherichia coli thermorésistant également désignées conformes fécaux, la culture de cellules peut être réalisée à une température de 43 °C à 45 °C.

Selon l'invention, la durée de l'étape b) de culture peut être comprise de de 20 à 48 heures, par exemple de 24 heures.

Dans la présente, la culture de cellules de l'étape b) peut être réalisée sous une atmosphère contrôlée à 5% de CO₂.

Selon l'invention, l'étape a) de culture de cellules peut être réalisée à une température comprise de 30 à 40°C, par exemple de 37°C.

Selon l'invention, l'étape a) peut être réalisée dans tout contenant de culture adapté connu de l'homme du métier. Il peut s'agir par exemple d'une flasque de 75 cm² et/ou de toute flasque connu de l'homme du métier permettant la culture des cellules. Il peut s'agit par exemple de plaques multi-puits, par exemple des plaques 6 puits, des plaques 12 puits, des plaques 24 puits, des plaques 48 puits et/ou des plaques 96 puits. Il peut s'agir par exemple d'un bioréacteur, par exemple comprenant un volume de milieu de culture de 50 mL à 100 L.

Selon l'invention, l'étape c) de récupération de vésicule extracellulaire peut être réalisé par tout procédé adapté connu de l'homme du métier. Il peut s'agir par exemple d'un procédé comprenant une étape de centrifugation, une étape de filtration, d'ultracentrifugation Par exemple, l'étape b) de récupération de vésicule extracellulaire peut comprendre
- une étape b' de récupération du milieu M2,
- une étape b" de centrifugation,
- une étape b‴ de récupération du surnageant obtenu à l'étape b" et de filtration dudit surnageant, et
- une étape bʺʺ d'ultracentrifugation, lavage du culot comprenant les vésicules extracellulaires par une solution et élimination de ladite solution de lavage.

Dans la présente, dans l'étape b', la récupération du milieu peut être réalisée par tout procédé connu de l'homme du métier adapté. Il peut s'agir par exemple d'une aspiration du milieu. Par exemple l'aspiration du milieu peut être réalisé par tout dispositif adapté connue de l'homme du métier, par exemple par une pipette stérile, par exemple de 10 mL. Dans la présente, le milieu aspiré peut être délivré dans tout contenant adapté, par exemple dans un tube, par exemple un tube de 50 mL.

Dans la présente, l'étape b" de centrifugation peut être réalisée par tout procédé connu de l'homme du métier. Il peut s'agir par exemple d'une centrifugation 2 000 à 10 000 x g, par exemple de 2000 à 10 000 g, par exemple de 10 000 g.

Selon l'invention, l'étape b" de centrifugation peut être réalisée pendant une durée de 5 à 40 min, par exemple de 20 à 30 minutes.

Dans la présente, dans l'étape b‴, la récupération du surnageant peut être réalisée par tout procédé connu de l'homme du métier adapté. Il peut s'agir par exemple d'une aspiration du milieu.Par exemple l'aspiration du milieu peut être réalisé par tout dispositif adapté connue de l'homme du métier, par exemple par une pipette stérile, par exemple de 10 mL. Dans la présente, le milieu aspiré peut être délivré dans tout contenant adapté, par exemple dans un tube, par exemple un tube de 50 mL.

Dans la présente, dans l'étape b‴, la filtration du surnageant peut être réalisée par tout procédé connu de l'homme du métier adapté. Il peut s'agir par exemple d'une filtration sur une membrane, par exemple une filtration avec une membrane avec un diamètre de pores compris de 0,20 à 0,30µm, par exemple égale à 0,22µm.

Dans la présente, dans l'étape bʺʺ la centrifugation peut être réalisée par tout procédé connu de l'homme du métier. Il peut s'agir d'une centrifugation selon le procédé décrit dans Xu, et al., 10.1080/08958378.2019.1597220 [40] ou Thery, et al., 10.1002/0471143030.cb0322s30 [41]. Il peut s'agir par exemple d'une centrifugation à une vitesse de 30 000 à 45 000 g, par exemple à une vitesse de 34 000 tours par minutes (100 000 x g). Dans l'étape bʺʺ la centrifugation peut être réalisée pendant une durée de 5 à 80 min, par exemple de 65 à 75 minutes, par exemple pendant 70 minutes.

Dans la présente, dans l'étape "b", le lavage du culot comprenant les vésicules extracellulaires peut être réalisé par tout procédé connu de l'homme du métier, par exemple, par aspersion, trempage du culot dans une solution de lavage.

Dans la présente, par solution de lavage on entend toute solution de lavage de vésicule extracellulaire connue de l'homme du métier. Il peut s'agir par exemple de tampon phosphate salin (PBS) à pH compris de 7 à 7,8, par exemple 7,4. Il peut s'agir également d'une solution tampon disponible dans le commerce par exemple un tampon phosphate salin (PBS) 1X commercialisée respectivement par la société Eurobio.

Dans la présente, dans l'étape bʺʺ l'élimination de la solution de lavage du culot peut être réalisée par tout procédé connu de l'homme du métier adapté. Il peut s'agir par exemple d'une centrifugation, par exemple une ultracentrifugation, à une vitesse 30 000 à 45 000 tours par minutes, par exemple à une vitesse de 34 000 tours par minutes (100 000 x g) pendant une durée de 5 à 80 min, par exemple de 65 à 75 minutes, par exemple pendant 70 minutes. Avantageusement, cette étape permet de culoter les vésicules extracellulaires lavées. La solution de lavage peut être ensuite éliminée par tout procédé adapté connu, par exemple par aspiration, par exemple avec une pipette stérile.

La présente invention a également pour objet une vésicule extracellulaire susceptible d'être obtenu par le procédé selon l'invention.

Avantageusement, la vésicule extracellulaire obtenu par le procédé selon l'invention comprend au moins un marqueur choisi dans le groupe comprenant CD9, CD63, CD81, ALIX et TSG101.

Avantageusement, les inventeurs ont démontré que la vésicule extracellulaire obtenue par le procédé selon l'invention comprend une augmentation significative de la concentration des protéines choisies dans le groupe comprenant la chromatine cible de prmt1 (« chromatin target of prmt1 » (Chtop)), l'inhibiteur de la voie du facteur de tissue (« tissue factor pathway inhibitor » (Tfpi)), la disintegrine et métalloprotéinase avec un motif 4 de thrombospondine de type 1 ( « disintegrin and metalloproteinase with thrombospondin type 1 motif 4 » Adamts4), la protéine angiogénique riche en cystéine 61 (« cysteine-rich angiogenic protein 61 » (Ccn1)) et du facteur neurotrophique dérivé des neurones (« neuron-derived neurotrophic factor » (Ndnf)).

Les inventeurs sont les premiers à avoir démontré que les vésicules extracellulaires obtenues par le procédé selon l'invention comprennent à leurs surfaces des polymères biocompatibles, lesdits polymères étant fixés sur les vésicules extracellulaires, par exemple par liaison à des protéines d'adhésions cellulaires ou des lipoprotéines membranaires, avec une affinité très forte. Les inventeurs ont également démontré de manière surprenante et inattendu que les vésicules extracellulaires obtenues lorsqu'elles sont utilisées avantageusement adresser jusqu'à la lésion tissulaire et/ou le site lésionnel. En d'autres termes, les vésicules extracellulaires combinées aux polymères biocompatibles selon l'invention, par exemple les vésicules extracellulaires sur lesquelles sont fixés des polymères biocompatibles selon l'invention, lorsqu'elles sont utilisées pour le traitement d'une lésion tissulaire ciblent spécifiquement ladite lésions et/ou sont spécifiquement adressées à ladite lésion.

La présente invention a également pour objet une vésicule extracellulaire susceptible d'être obtenue selon le procédé de l'invention pour son utilisation dans la prévention et/ou dans le traitement d'une lésion tissulaire.

La lésion tissulaire est telle que définie ci-dessus.

### Brève description des figures

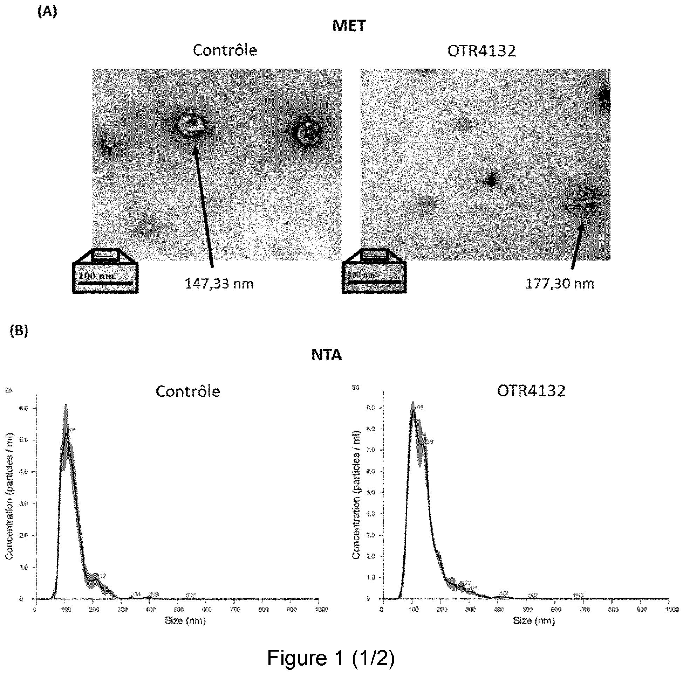

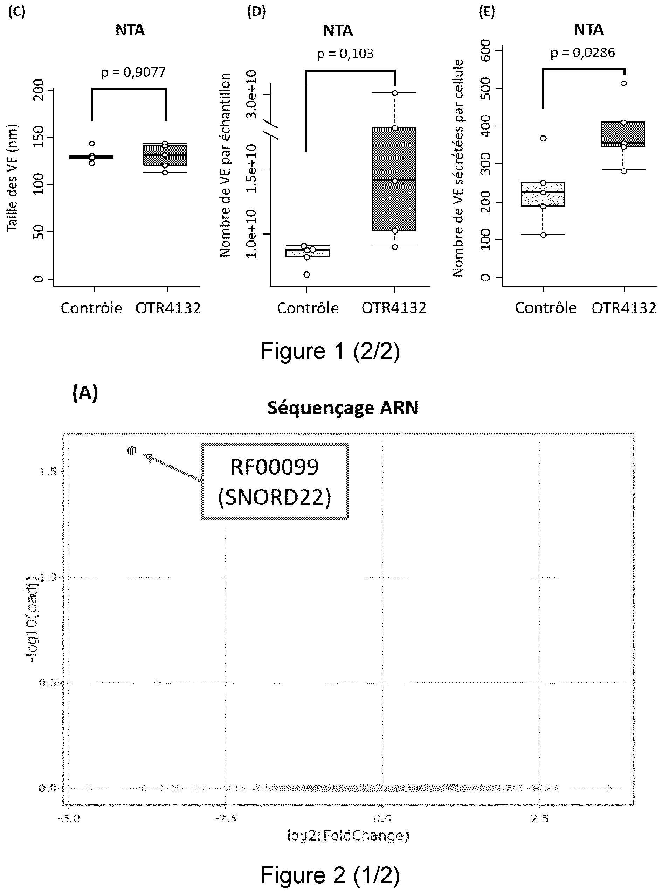

La figure 1 représente des photographies et courbes de vésicules extracellulaires. La figure 1A représente des photographies microscopie électronique à transmission après coloration négative avec 1,5% d'acétate d'uranyle. La figure1 B représente une courbe de la concentration des particules (particules / ml) (ordonnée) présentes dans le milieu obtenu en fonction de leur taille (nm) (abscisse) en fonction de la culture des cellules souches mésenchymateuses (MSC) sans (control) ou en présence de polymère biocompatible, à savoir l'OTR4132. La figure 1C représente un diagramme correspondant à la taille des vésicules extracellulaires (ordonné) obtenues après culture des cellules souches mésenchymateuses (MSC) sans (control) ou en présence de polymère biocompatible, à savoir l'OTR4132 le nombre de VE. La figure 1D représente un diagramme correspondant au nombre de vésicules extracellulaires obtenues (ordonné) après culture des cellules souches mésenchymateuses (MSC) sans (control) ou en présence de polymère biocompatible, à savoir l'OTR4132. Figure 1E représente un diagramme correspondant au nombre de vésicules extracellulaires par cellules (ordonné) obtenues après culture des cellules souches mésenchymateuses (MSC) sans (control) ou en présence de polymère biocompatible, à savoir l'OTR4132.

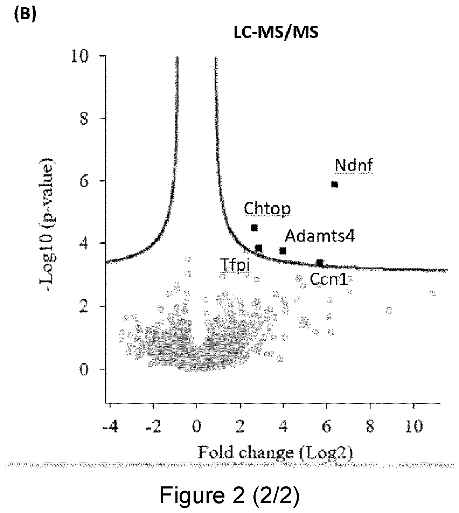

La figure 2A représentant un diagramme de volcan (Volcano plot) de l'expression de 500 gènes les plus variables, l'ordonné correspond au logarithme négatif en base 10 de la valeur de p calculée. l'abscisse correspond à la variation en logarithme de base 2 de l'expression des transcrits en ARN messager observée entre les deux échantillons (La figure 2B représentant un diagramme de volcan (Volcano plot) de la concentration de protéines dans les vésicules extracellulaires obtenues après culture des MSC en présence ou non d'un polymère biocompatible, à savoir l'OTR4132, l'ordonné correspond au logarithme négatif en base 10 de la valeur de p calculée l'abscisse correspond à la variation en logarithme de base 2 de l'enrichissement protéique observé entre les deux échantillons.

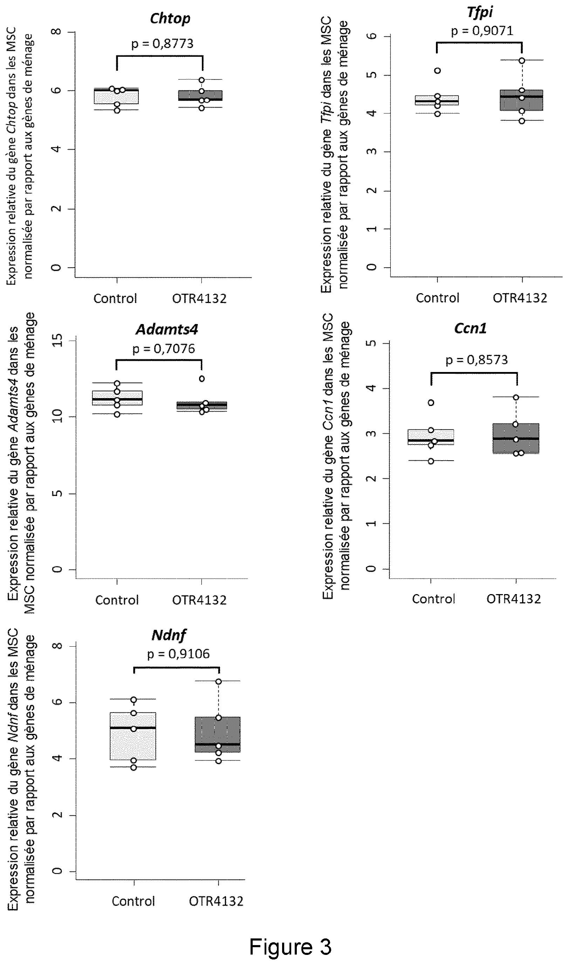

La figure 3 représente les niveaux d'expressions relatives des gènes Chtop, Tfpi, Adamts4, Ccn1 et Ndnf dans les MSC en fonction de leur culture dans un milieu sans ou avec l'OTR4132. Les valeurs sont représentées sous forme de médiane +/- IQ calculées à partir de 5 expériences indépendantes (test-t de Student). L'ordonné représente Expression relative du gène dans les MSC normalisée par rapport aux gènes de ménage.

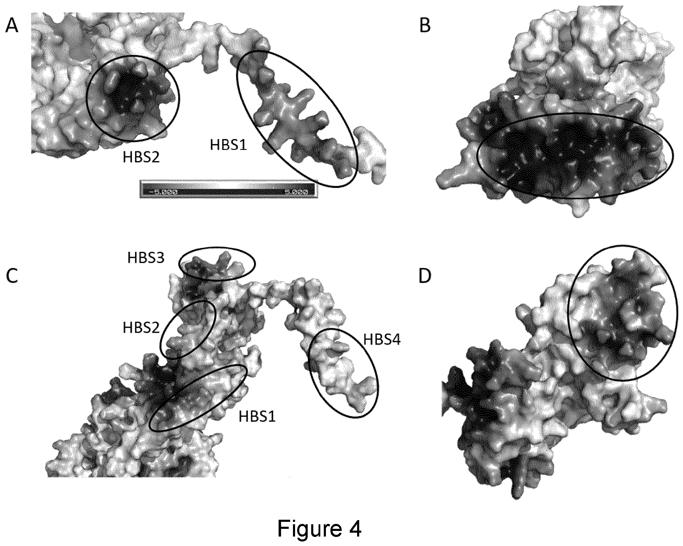

La figure 4 représente une représentation schématique des protéines Tfpi (Figure 4A), Ccn1 (Figure 4B), Adamts4 (Figure 4C) et Ndnf (Figure 4D). Les sites de liaison à l'héparine dans Tfpi, l'un dans le domaine C-terminal désordonné qui comprend un regroupement ou cluster de résidus basiques (HBS1), et le second dans le 3eme domaine de type BPTI/Kunitz inhibitor (HBS2). HBS : heparan-binding site ou site de liaison au heparan, et le chiffre correspond à la numérotation du site.

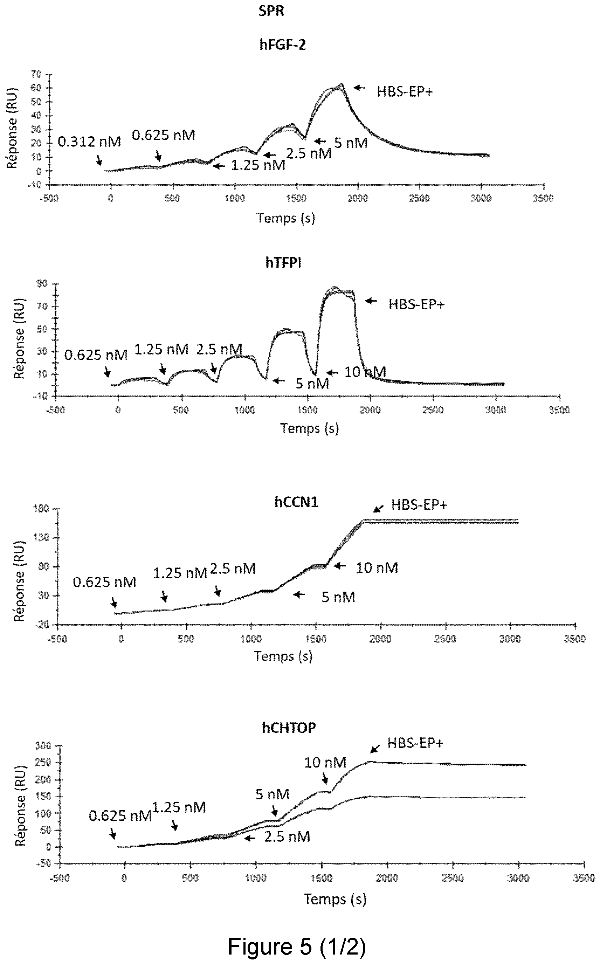

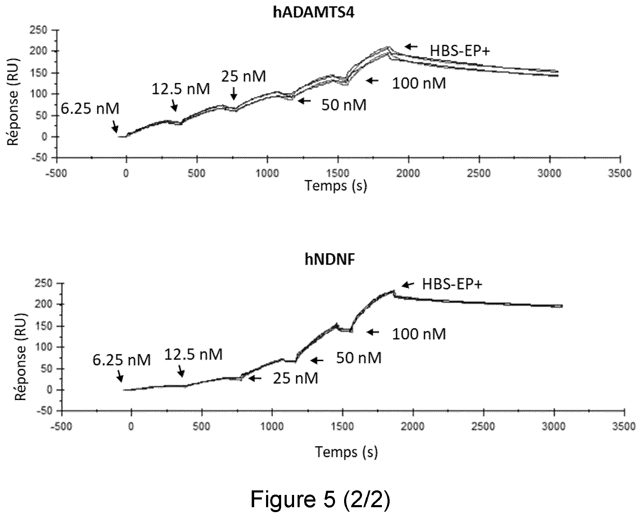

La figure 5 représente les sensogrammes représentent la réponse de résonance plasmonique de surface (SPR) en RU en fonction du temps en seconde (s) pour les 5 protéines : Facteur de croissance de base des fibroblastes humains 2 (« Human Basic Fibroblast Growth Factor 2» (hFGF2)), inhibiteur de la voie du facteur tissulaire (hTFPI), protéine angiogénique riche en cystéine 61 (« cysteine-rich angiogenic protein 61 » hCCN1), la chromatine cible de prmt1 :(« chromatin target of prmt1 » hCHTOP), la disintégrine et métalloprotéinase avec un motif 4 de thrombospondine de type 1 ( « disintegrin and metalloproteinase with thrombospondin type 1 motif 4 » hADAMTS44, le facteur neurotrophique dérivé des neurones humain (« neuron-derived neurotrophic factor » hNDNF) et le contrôle positif FGF-2.

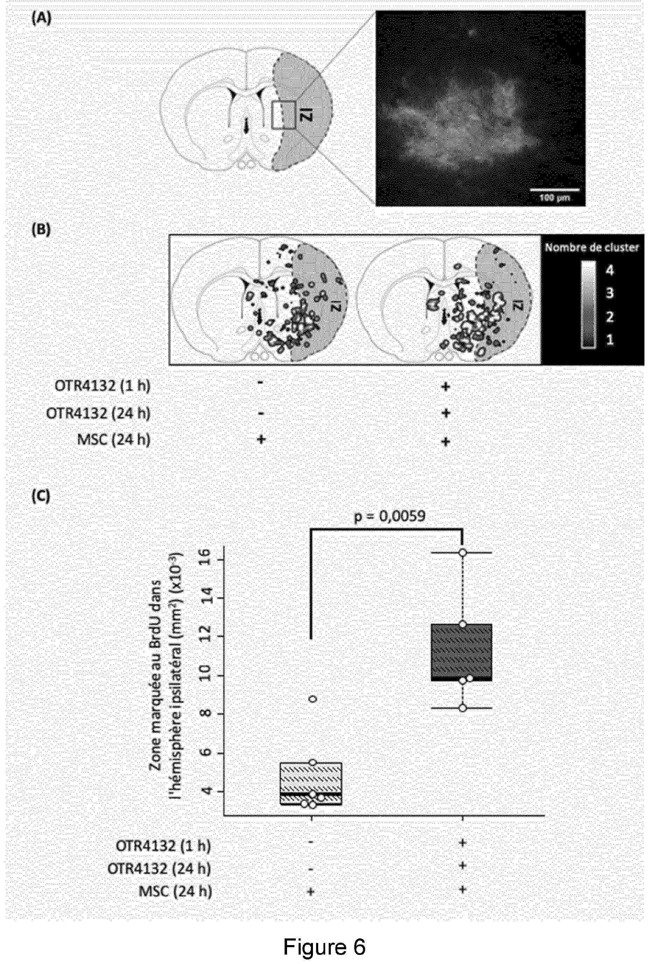

La figure 6 représente l'effet d'un polymère biocompatible, à savoir l'OTR4132 sur la localisation des cellules MSC dans une zone ischémiée du cerveau. La figure 6A représente des images en microscopie optique (Leica microsystems DMi8 et diagramme après traitement avec le logiciel ImageJ (marque de commerce) (Wayne Rasband, NIMH, Maryland, USA) (Schneider, Rasband et Eliceiri, 2012). La figure 6A représente une image de regroupement de MSC dans l'hémisphère ipsilatéral proche du site lésionnel. La figure 6B représente une image de regroupement de MSC dans l'hémisphère ipsilatéral proche du site lésionnel après administration à différent temps de l'OTR4132 et/ou de cellules MSC 6B. La figure 6C représente un diagramme représentant la surface en mm² marquée au BrdU dans l'hémisphère ipsilatérale (ordonné) mesurée par immunohistochimie en fonction de l'administration de MSC en combinaison ou non avec le polymère biocompatible OTR4132 à différent temps après la lésion.
Figure 7. Représentations (Figures 7A et 7B) en 3 dimensions de la structure tertiaire du collagène 7A humain selon deux perspectives de vues montrant les zones riches en acides aminés basiques et dont la charge est globalement positive et les zones riches en acides aminés acides dont la charge est globalement négative à pH physiologique. Zones chargées positivement représentées par des cercles noirs sur la figure.

D'autres avantages pourront encore apparaître à l'Homme du métier à la lecture des exemples ci-dessous, illustrés par les figures annexées, donnés à titre illustratif.

### Exemples

### Exemple 1: Composition comprenant des polymères biocompatibles et des Vésicules Extracellulaires

### A/ Préparation des polymères biocompatibles.

La synthèse des polymères biocompatibles, RGTA, est décrite dans l'art antérieur, par exemple dans le brevet US7396923 intitulé « procédé de sulfonation de composés comprenant des groupements hydroxyles (OH) libres ou des amines primaires ou secondaires » [15] et également dans la référence bibliographique Yasunori I. et al., Biomaterials 2011, 32 :769e776) et Petit E. et al,. Biomacromolecules. 2004 Mar-Apr; 5(2):445-52 [16].

Plusieurs RGTA sont connus et décrits ont été utilisés dont le OTR4120 décrit de nombreuses publications précliniques et cliniques (RGTA^{®}-based matrix therapy - A new branch of regenerative medicine in locomotion. Barritault D, Desgranges P, Meddahi-Pellé A, Denoix JM, Saffar JL. Joint Bone Spine. 2017 May;84(3):283-292. doi: 10.1016/j.jbspin.2016.06.012 ([4], Barritault, et al., 10.1016/j.jbspin.2016.06.012), RGTA@ or ReGeneraTing Agents mimic heparan sulfate in regenerative medicine: from concept to curing patients. Barritault D, Gilbert-Sirieix M, Rice KL, Siñeriz F, Papy-Garcia D, Baudouin C, Desgranges P, Zakine G, Saffar JL, van Neck J. Glycoconj J. 2017 Jun;34(3):325-338. doi: 10.1007/s10719-016-9744-5 ([3], Barritault, et al., 10.1007/s10719-016-9744-5).

Le composé OTR4131 est un composé comprenant un radical Z qui est un acide gras, à savoir l'acide acétique tel que décrit dans Frescaline G. et al., Tissue Eng Part A. 2013 Jul;19(13-14):1641-53. doi: 10.1089/ten.TEA.2012.0377 ([42], Frescaline, et al., 10.1089/ten.TEA.2012.0377), Randomized controlled trial demonstrates the benefit of RGTA^{®} based matrix therapy to treat tendinopathies in racing horses. Jacquet-Guibon S, Dupays AG, Coudry V, Crevier-Denoix N, Leroy S, Siñeriz F, Chiappini F, Barritault D, Denoix JM. PLoS One. 2018 Mar 9;13(3):e0191796. doi: 10.1371/journal.pone.0191796 ([43], Jacquet-Guibon, et al., 10.1371/journal.pone.0191796). D'autres composés également décrits dans les documents brevets US06689741 [44], US2014301972A1 [45] dans lequel Z est un acide aminé comme la phénylalanine (Heparan sulfate proteoglycans médiate internalization and propagation of spécifie proteopathic seeds. Holmes BB, DeVos SL, Kfoury N, Li M, Jacks R, Yanamandra K, Ouidja MO, Brodsky FM, Marasa J, Bagchi DP, Kotzbauer PT, Miller TM, Papy-Garcia D, Diamond MI. Proc Natl Acad Sci U S A. 2013 Aug 13;110(33):E3138-47. doi: 10.1073/pnas.1301440110 ([46], Holmes, et al., 10.1073/pnas. 1301440110)) ou autre composé hydrophobe (Structure-activity studies of heparan mimetic polyanions for anti-prion thérapies. Ouidja MO, Petit E, Kerros ME, Ikeda Y, Morin C, Carpentier G, Barritault D, Brugère-Picoux J, Deslys JP, Adjou K, Papy-Garcia D. Biochem Biophys Res Commun. 2007 Nov 9;363(1):95-100 ([47], Ouidja, et al., 10.1016/j.bbrc.2007.08.113)).

### B/ Préparation des vésicules extracellulaires

Dans cet exemple les cellules utilisées étaient des cellules souches mésenchymateuses (MSC) extraites des fémurs et tibia de rats Sprague-Dawley, rats mâles âgés de 8 à 10 semaines, Janvier Labs, France. Les animaux ont été anesthésiés par inhalation d'isoflurane 5 % dans un mélange O₂/N₂O (ratio 1/3) puis mis à mort par inhalation au CO₂. Après la dissection des fémurs et des tibias sous une hotte à flux laminaire, ceux-ci ont été placés dans un bain d'alcool, suivi d'un bain de pénicilline-streptomycine, puis rincés dans une solution saline 1X de tampon. Les épiphyses ont été coupées et la moelle osseuse a été récupérée dans un milieu de culture Dulbecco's Modified Eagle's Medium (DMEM) - low glucose (référence commerciale D4655, commercialisé par la société Sigma-Aldrich) par des allers-retours dans la lumière osseuse à l'aide d'une aiguille de 21 Gauge montée sur une seringue de 5 mL contenant 3 mL de milieu de culture. Le contenu récupéré a été ensuite centrifugé 5 min à 300 x g. Les culots cellulaires ont été re-suspendus dans 10 mL de milieu de culture Dulbecco's Modified Eagle's Medium (DMEM) - low glucose (référence commerciale D4655, commercialisé par la société Sigma-Aldrich), supplémenté de 1 % de pénicilline-streptomycine (référence commerciale P4333, commercialisé par la société Sigma-Aldrich), de 2 mM de L-glutamine (référence commerciale G7513, commercialisé par la société Sigma-Aldrich) et de 20 % de sérum de veau foetal (référence commerciale FB-1001, commercialisé par la société Biosera). Les cellules ont été ainsi mises en culture dans des boîtes de culture de 75 cm² (T75). Les cellules ont été incubées sous atmosphère humide composée de 95 % d'air et de 5 % de CO₂ à 37°C. Après 3 à 4 jours, les boîtes de culture de 75 cm² (T75) ont été délicatement secouées, rincées 2 fois avec une solution de Tampon Phosphate Salin (PBS) puis le milieu a été remplacé par du milieu complet similaire à celui utilisé précédemment, éliminant ainsi les cellules hématopoïétiques non adhérentes, les cellules mortes et les débris cellulaires contrairement aux MSC qui ont adhérées au fond des T75. Le milieu a été ainsi changé tous les 3 à 4 jours et les cellules souches mésenchymateuses (MSC) ont été décollées par trypsination du tapis cellulaire passées lorsqu'elles atteignent 90 % de confluence, lavées et diluées afin d'être réensemencées à une densité cellulaire de 1.10⁶ cellules dans une nouvelle flasque de 75 cm² T75 dans un milieu de culture Dulbecco's Modified Eagle's Medium (DMEM) - low glucose (référence commerciale D4655, commercialisé par la société Sigma-Aldrich), supplémenté de 1 % de pénicilline-streptomycine (référence commerciale P4333, commercialisé par la société Sigma-Aldrich), de 2 mM de L-glutamine (référence commerciale G7513, commercialisé par la société Sigma-Aldrich) et de 10 % de sérum de veau foetal (référence commerciale FB-1001, commercialisé par la société Biosera).

Des MSC ont été maintenues en culture au 3^{ème} passage jusqu'à ce qu'elles atteignent 80 % de confluence dans des flasques T75, celles-ci ont été lavées. 0,1 µg/mL de polymère biocompatible, à savoir d'OTR4132, ont été ajouté ou non au milieu de culture, ledit milieu était déplété en sérum de veau foetal (SVF). Après 24 h de culture dans le milieu comprenant de l'OTR4132, 240 mL de milieu conditionné par condition (sans ou avec 0,1 µg/mL d'OTR4132) provenant de la culture de 24 T75 de MSC ont été collectés et centrifugés 2 fois à 4 °C : 2 000 × g pendant 20 min et 10 000 × g pendant 30 min, pour éliminer les cellules mortes et les débris cellulaires. Le surnagent a été ensuite filtré sur une membrane de 0,22 µm (Sarstedt) et centrifugé à 100 000 × g (34 000 tours par minutes) (Beckman Coulter) pendant 70 min à 4 °C selon le procédé décrit dans Théry et al., 2006, et Xu et al., 2019, ([41], Thery, et al., 10.1002/0471143030.cb0322s30) ([40], Xu, et al., 10.1080/08958378.2019.1597220). Le culot a été lavé avec du Tampon Phosphate Salin (référence commerciale CS1PBS01K BP, commercialisé par la société Eurobio) PBS et soumis à une seconde ultracentrifugation à 100 000 × g (34 000 tours par minutes, Beckman Coulter) pendant 70 min à 4°C. Le culot ainsi obtenu comprend les vésicules extracellulaires avec ou sans polymère biocompatible qui ont été remises en suspension dans du Tampon Phosphate Salin (référence commerciale CS1PBS01K BP, commercialisé par la société Eurobio). PBS 1X et stockées à -80 °C pour des analyses ultérieures. Les solutions ainsi obtenues comprenaient lesdites vésicules extracellulaires.

Une étude des vésicules extracellulaires (VE) ainsi obtenues a été réalisées. Ainsi, une évaluation des VE obtenues de MSC cultivées en présence d'OTR4132 étaient différentes de celles provenant de MSC cultivées sans OTR4132, les différents paramètres suivants ont été étudiés sur les VE isolées :
- leur quantité et leur taille, à savoir le diamètre moyen, en utilisant un analyseur de vésicules (NanoSight NS300) et la microscopie électronique ;
- leur contenu en matériel génétique en utilisant la technique de RNAseq ;
- leur contenu protéique et leur interaction avec l'OTR4132 en utilisant ;
   - la protéomique ;
   - l'identification in silico de sites de liaison à l'OTR4132 ;
   - la résonance plasmonique de surface (SPR).

La figure 1 représente les différents résultats obtenus. La figure 1A représente des photographies en microscopie électronique à transmission (MET). 4 µL de VE pures ont été déposés sur une grille de carbone/formvar/cuivre, incubés 5 min à température ambiante, à savoir 20°C, et séchés par capillarité sur du papier whatman. Afin de fixer et colorer les VE, une goutte d'acétate d'uranyle à 1,5 % (Référence fournisseur 73943-25G Honeywell Fluka 25GR, Thermo Fischer Scientific) a été déposée sur la grille, laissée incuber 15 sec à température ambiante, à savoir 20 °C, et de nouveau séchée par capillarité sur du papier whatman. Les VE ont ensuite été visualisées à l'aide d'un microscope électronique à transmission de type JEOL JEM 1011 TEM 100kV (JEOL USA, Inc, Peabody, MA) avec une caméra CCD Orius SC200 (Gatan, USA). Tel que démontré sur ces photographies, les vésicules extracellulaires obtenues ont un diamètre proche : 147,33 nm et 177,30 nm respectivement. Les concentrations des échantillons et les tailles de VE ont été mesurées à l'aide d'un NanoSight NS300 (Malvern Panalytical) avec un laser de 405 nm. Les échantillons ont été dilués dans du PBS 1X (Référence fournisseur 11503387Gibco^{™} PBS, pH de 7,4, FisherScientific) pour obtenir des concentrations acceptables selon les recommandations d'utilisation de l'appareil par le fabricant à savoir de 3.10⁸ à 5.10⁸ VE/mL. Un rinçage au PBS 1X de l'appareil a été réalisé entre chaque analyse. Le logiciel NTA 3.3 (Malvern Panalytical) a été utilisé pour traiter et analyser les 5 vidéos de 60 secondes enregistrées par échantillon analysé au NanoSight NS300. La figure 1B représente des diagrammes de la concentration des particules (particules / ml) présentes dans le milieu obtenu en fonction de leur taille (nm) en fonction de la culture des cellules souches mésenchymateuses (MSC) sans (control) ou en présence de polymère biocompatible, à savoir l'OTR4132. Tel que démontré, les échantillons comprenaient des VE avec un pic de taille autour de 100 à 150 nm, caractéristique des exosomes. La figure 1C représente un diagramme correspondant à la taille des vésicules extracellulaires obtenues après culture des cellules souches mésenchymateuses (MSC) sans (control) ou en présence de polymère biocompatible, à savoir l'OTR4132. Tel que démontré, l'OTR4132 dans le milieu de culture ne modifie pas la taille des VE obtenues par des MSC avec une taille médiane dans les 2 conditions aux alentours de 130 nm. La figure 1D représente un diagramme correspondant au nombre de vésicules extracellulaires obtenues après culture des cellules souches mésenchymateuses (MSC) sans (control) ou en présence de polymère biocompatible, à savoir l'OTR4132. Tel que démontré, l'OTR4132 augmente le nombre de VE présentes dans les échantillons. La figure 1E représente un diagramme correspondant au nombre de vésicules extracellulaires par cellules obtenues après culture des cellules souches mésenchymateuses (MSC) sans (control) ou en présence de polymère biocompatible, à savoir l'OTR4132. Tel que démontré, la présence de l'OTR4132 dans le milieu de culture augmente significativement le nombre de VE sécrétées/obtenues à partir de MSC.

Les résultats obtenus démontrent clairement qu'aucune différence dans le nombre de MSC n'a été observé en fonction des conditions de culture (en présence ou absence d'OTR4132). Les résultats démontrent également clairement que le nombre de Vésicules Extracellulaires obtenues était deux fois supérieur en présence d'OTR4132 sans modification de la taille et/ou de la morphologie desdites Vésicules Extracellulaire (Figure 1 A et B).

La Figure 1 E ci-dessous illustre cette différence significative dans le nombre de VE/cellules. (t-test de Student, p = 0,0286).

Une analyse protéomique des vésicules extracellulaires obtenues a été également réalisées par spectrométrie de masse. Afin de digérer les échantillons, ces derniers ont été incubés en présence de 20 % de SDS (Référence fournisseur 436143, Sigma-Aldrich, Merck) à une concentration finale de 5 %, réduits avec 20 mM de TCEP (chlorhydrate de tris (2-carboxyéthyl) phosphine) (Référence fournisseur : C4706, Sigma-Aldrich, Sigma-Aldrich, Merck,) et alkylés avec 50 mM de CAA (chloracétamide) (Référence forunisseur: 22790, Sigma-Aldrich, Merc) pendant 5 min à 95 °C. De l'acide phosphorique aqueux (Référence fournisseur 1.00573, Sigma-Aldrich, Merck,) a été ajouté à une concentration finale de 2,5 %, suivi de l'ajout de tampon de liaison S-Trap (90 % de méthanol aqueux, 100 mM TEAB, pH 7,1) Références fournisseur 34860 et 241059, Sigma-Aldrich, Merck. Les mélanges ont ensuite été chargés sur les colonnes S-Trap^{™} (commercialisé par la société ProtiFi, USA). Cinq lavages ont été effectués pour une élimination complète du SDS. Les échantillons ont été digérés avec 1,5 µg de trypsine (Promega) à 47 °C pendant 1 h. Après élution, les peptides ont été séchés sous vide et remis en suspension dans 2 % d'Acetonitrile (ACN) (Référence fournisseur 439134, Sigma-Aldrich, Merck), 0,1 % d'acide formique (Référence fournisseur W248703, Sigma-Aldrich, Merck dans de l'eau à chromatographie en phase liquide à haute performance (ou « high performance liquid chromatography », (HPLC)) avant analyse en spectrométrie de masse. Les peptides tryptiques ont été remis en suspension dans 30 µL et le volume correspondant à 400 ng pour chaque échantillon a été injecté sur un système HPLC (Bruker Daltonics, Allemagne) couplé à un spectromètre de masse timsTOF Pro (Bruker Daltonics, Allemagne). La séparation HPLC (solvant A : 0,1 % d'acide formique dans l'eau, 2 % d'acétonitrile ; solvant B : 0,1 % d'acide formique dans l'ACN) a été effectuée à 250 nL/min à l'aide d'une colonne à émetteur (C18, 25 cm × 75 µm × 1,6 µm) (Ion Optics, Australie) en utilisant un gradient d'élution de 40 min (2 à 11 % de solvant B pendant 19 min ; 11 à 16 % pendant 7 min ; 16 % à 25 % pendant 4 min ; 25 % à 80 % pendant 3 min et enfin 80 % pendant 7 min pour laver la colonne). Les données de spectrométrie de masse ont été acquises à l'aide de la méthode d'acquisition de fragmentation en série par accumulation parallèle (PASEF (marque déposée)) avec un mode d'acquisition dépendant des données (ou « data dépendent acquisition », DDA). Les mesures ont été effectuées avec un rapport masse/charge de 100 à 1700 thomson (Th). Les données obtenues ont été analysées à l'aide de la version 2.0.1.0 du logiciel MaxQuant et recherchées avec le moteur de recherche Andromeda dans la base de données UniProtKB/Swiss-Prot Rattus norvegicus mélangée à ses entrées TrEMBL (mise à jour en février 2021, 48085 entrées). Les scores ont été calculés dans MaxQuant comme décrit précédemment ([48], Cox, et al., 10.1038/nbt.1511) . Des analyses statistiques et bio-informatiques, y compris des cartes thermiques, des tracés de profil et des regroupements, ont été réalisées avec le logiciel Perseus (version 1.6.14.0, Max Quant). Pour la comparaison statistique, 2 groupes ont été définis à savoir avec ou sans OTR4132, traités et non traités, contenant chacun 3 réplicats biologiques. Un test T de Student a été réalisé et l'erreur de première espèce (α) a été fixée à 0,05. Les résultats de cette analyse ont démontré que les vésicules extracellulaires obtenues, quel que soit les conditions de culture des MSC exprimaient les marqueurs suivants CD9, CD63, CD81, ALIX et TSG101 qui sont spécifiques aux exosomes. En outre, tel que démontré sur la figure 2A représentant une représentation en forme de volcan (Volcano plot) concernant l'expression de 500 gènes les plus variables, aucune différence d'expression n'a été identifiée en fonction de la culture en présence ou en l'absence d'un polymère biocompatible, à savoir l'OTR4132.

Toutefois, tel que démontré sur la Figure 2B et le tableau 1 ci-dessous représentant respectivement un diagramme en présentation en Volcan et un tableau de la concentration de protéines dans les vésicules extracellulaires obtenues après culture des MSC en présence ou non d'un polymère biocompatible, à savoir l'OTR4132.

**Tableau 1 : Facteur de variation en log base 2.**

| **Protéines** | **Log2 (fold change)** | **p-value (t-test de Student)** | |
|---|---|---|---|
| **Chtop** | 2,61246 | | 3,22.10⁻⁵ |
| **Tfpi** | 2,78528 | | 0,0001 |
| **Adamts4** | 3,89914 | | 0,0001 |
| **Ccn1** | 5,63462 | | 0,0004 |
| **Ndnf** | 6,33113 | | 1,34.10⁻⁶ |

Tel que démontré, en présence d'OTR4132 une augmentation significative de la concentration dans les vésicules extracellulaire de la chromatine cible de prmt1 (« chromatin target of prmt1 » (Chtop)), l'inhibiteur de la voie du facteur de tissue (« tissue factor pathway inhibitor » (Tfpi)), la disintegrine et métalloprotéinase avec un motif 4 de thrombospondine de type 1 ( « disintegrin and metalloproteinase with thrombospondin type 1 motif 4 » Adamts4), la protéine angiogénique riche en cystéine 61 (« cysteine-rich angiogenic protein 61 » (Ccn1)) et du facteur neurotrophique dérivé des neurones (« neuron-derived neurotrophic factor » (Ndnf)) a été observée. En particulier, l'augmentation de la concentration dans les vésicules extracellulaire de la Chtop, du Tfpi, de l'Adamts4, de la Ccn1 et de Ndnf était respectivement de 6, 7, 15, 50 et 80 fois dans les vésicules extracellulaires, à avoir des exosomes, tel que démontré dans la figure 2B.

Une analyse de l'expression dans les MSC des gènes *Chtop,* du *Tfpi,* la *Adamts4, Ccn1* et *Ndnf* a été également réalisée. Ainsi, l'ARNm des MSC à partir desquels les VE ont été isolées a été extrait et purifié à l'aide du kit RNeasy Micro (Référence fournisseur 74104, Qiagen, Courtaboeuf, France). La concentration d'ARNm a été mesurée avec le spectrophotomètre NanoDrop^{™} (marque de commerce) 2000 (Référence fournisseur ND-2000, Thermo Fisher Scientific, USA). Les ARNm ont ensuite été rétro-transcrits avec le Supermix de transcription inverse iScript (Bio-Rad), et l'ADNc a été amplifié avec le système de détection PCR en temps réel CFX96 Touch (Bio-Rad) et le Supermix iTaq Universal SYBR^{®} Green (Bio-Rad) avec des amorces conçues par Eurogentec (Cf tableau 2 ci-dessous). La glycéraldéhyde-3-phosphate déshydrogénase (Gapdh), la cyclophiline A et l'actine (Act) ont été utilisées comme contrôle d'ARN endogène (gènes domestiques). Les séquences d'amorce pour les gènes neuron-derived neurotrophic factor (*Ndnf*), chromatin target of prmt1 *(Chtop),* tissue factor pathway inhibitor *(Tfpi),* disintegrin and metalloproteinase with thrombospondin type 1 motif 4 *(Adamts4)* et cysteine-rich angiogenic protein 61 *(Ccn1)* ont été achetées chez Eurogentec décrites dans le tableau 2 ci-dessous.

**Tableau 2 : Séquences des amorces permettant l'analyse de l'expression des gènes de ménage et des gènes d'intérêt codant pour les protéines identifiées dans les vésicules extracellulaires de cellules stromales mésenchymateuses hématopoïétiques traitées avec l'OTR4132.**

| **Gènes** | **Catégories** | **Amorces** | **SEQ ID NO** |
|---|---|---|---|
| *Gapdh* | Gènes de ménage | F=5' CATCAAGAAGGTGGTGAAGC 3' | 10 |
| | | R=5' ACCACCCTGTTGCTGTAG 3' | 11 |
| *Cyclophiline A* | | F=5' CTAAGGCCAACCGTGAAAAG 3' | 12 |
| | | R=5' TACATGGTCGGGGTCTTGA 3' | 13 |
| *Act* | | F=5' GGGGAGAAAGGATTTGGCTA 3' | 14 |
| | | R=5' ACATGCTTGCCATCCAGCC 3' | 15 |
| *Chtop* | Gènes d'intérêt codant pour les 5 protéines identifiées dans les vésicules extracellulaires de cellules stromales mésenchymateuses hématopoïétiques traitées avec l'OTR4132. | F=5' CTTAAAGCAGCGCCTGGGTA 3' | 16 |
| | | R=5' CACCCCTAAGCAGGGTTCTG 3' | 17 |
| *Tfpi* | | F=5' TTAATGCTCTGCCCGAGGAAG 3' | 18 |
| | | R=5' AACCTCGGCAGATTCCAGGAT 3' | 19 |
| *Adamts4* | | F=5' GGCTGCTGTACCGATTACCA 3' | 20 |
| | | R=5' TCTACTCAGCGAAGCGAAGC 3' | 21 |
| *Ccn1* | | F=5` GTGCCGCCTGGTGAAAGAGA 3' | 22 |
| | | R=5' GCTGCATTTCTTGCCCTTTTTTAG 3' | 23 |
| *Ndnf* | | F=5′ GAGGAAGATAACACCCCACTGT 3′ | 24 |
| | | R=5' CCTGACCCATCTGCACTGGA 3' | 25 |
| *Hpse* | | F=5' CGGTTCTGACGGACTGCTT 3' | 26 |
| | | R=5' AAAACCCATAGGAAAAGGCG 3' | 27 |

Les gènes mentionnés dans le tableau 2 ci-dessus se rapporte aux gènes chez le rat.

Les échantillons ont été analysés en triplicata avec le protocole d'amplification suivant : 95 °C pendant 3 min suivi de 40 cycles à 95 °C pendant 3 sec et à 60 °C pendant 30 sec. Les niveaux d'expression ont été calculés à l'aide de la méthode Ct comparative (ΔCt) après normalisation avec les gènes domestiques *Gapdh, l'Act* et la *cyclophiline A.* Les résultats sont exprimés sous forme d'expression génique relative normalisée à la moyenne des gènes de ménage (*cyclophiline A, actine* et *GAPDH).* La figure 3 représente les niveaux d'expressions relatives des gènes dans les MSC en fonction de leur culture dans un milieu sans ou avec l'OTR4132. Les valeurs sont représentées sous forme de médiane +/- IQ calculées à partir de 5 expériences indépendantes (test-t de Student). Tel que démontré sur la figure 3, les résultats obtenus démontrent clairement aucune différence d'expression en fonction des conditions de culture des MSC.

Ces résultats démontrent clairement que la combinaison de vésicules extracellulaire et d'un exemple de polymère biocompatible permet avantageusement de protéger les protéines chromatine cible de prmt1 (« chromatin target of prmt1 » (Chtop)), l'inhibiteur de la voie du facteur de tissue (« tissue factor pathway inhibitor » (Tfpi)), la disintegrine et métalloprotéinase avec un motif 4 de thrombospondine de type 1 ( « disintegrin and metalloproteinase with thrombospondin type 1 motif 4 » Adamts4), la protéine angiogénique riche en cystéine 61 (« cysteine-rich angiogenic protein 61 » (Ccn1)) et le facteur neurotrophique dérivé des neurones (« neuron-derived neurotrophic factor » (Ndnf)).

Les protéines tfpi, ccn1 et adamts4 sont connues pour avoir des sites de liaison à l'héparine tel que représenté sur la Figure 4. Deux sites de liaison à l'héparine ont ainsi été décrits dans tfpi, l'un dans le domaine C-terminal désordonné qui comprend un cluster de résidus basiques (HBS1), et le second dans le 3eme domaine de type BPTI/Kunitz inhibitor (HBS2), identifié par comparaison des structures RMN de ce domaine avec ou sans héparine ([49], Mine, et al., 10.1021/bi011299g). Un site de liaison à l'héparine a été décrit dans ccn1 dans son domaine à noeuds cystéine qui possède un cluster de résidus basiques ([50], Chen, et al., 10.1074/jbc.M003040200). Quatre sites de liaison à l'héparine ont été décrits dans adamts4. Le site décrit dans le domaine thrombospondine de type I a été identifié par homologie avec le site de liaison à l'héparine trouvé dans adamts1 (HBS1). Trois sites de liaison à l'héparine ont également été identifiés dans le domaine enrichie en cystéine (HBS2) et dans le domaine spacer (HBS3 et HBS4), des peptides correspondant à leur séquence inhibent en effet la liaison à l'héparine de adamts4 ([51], Jones, et al., 10.1186/ar1783). Aucun site de liaison à l'héparine n'a été décrit dans Ndnf et Chtop.

Une analyse de l'affinité des protéines chromatin target of prmt1 (Chtop), le tissue factor pathway inhibitor (Tfpi), la disintegrin and metalloproteinase with thrombospondin type 1 motif 4 (Adamts4), la cysteine-rich angiogenic protein 61 (Ccn1) et le neuron-derived neurotrophic factor (Ndnf) et d'un polymère biocompatible, par exemple OTR4132, a été réalisée. Ainsi, la détermination de l'affinité a été réalisée par les techniques de Résonnance Plasmonique de Surface (SPR) sur un appareil BiacoreTM T200 (Cytiva) à 25 °C. Pour se faire, de la biotine a été greffée sur la molécule d'OTR4132 en utilisant l'aldéhyde libre en bout de chaine glycosidique selon le procédé décrit ci-après. La méthode choisie pour greffer une biotine sur le RGTA^{®} est une addition-élimination initiée par l'azote de l'acylhydrazide de la biotine à l'extrémité réductrice de la chaîne d'OTR4132. Un acylhydrazone liée (R-CO-NH-N=R') a été créée par le doublet d'électrons non participants (CH=N:) et un atome d'azote aminé à caractère acide (-NH-)[ 10.3390/molecules27248719]. Un excès d'acylhydrazide de biotine a été utilisé par rapport aux aldéhydes terminaux libres de l'OTR4132. L'OTR4132-biotinylé a été capturé sur plusieurs surfaces d'une puce de détection SA série S via son interaction avec la streptavidine préalablement fixée sur la puce BiacoreTM (SA, Série S, Cytiva). Pour mesurer la cinétique et les affinités d'interaction, des échantillons d'analytes correspondant aux 5 protéines (hTFPI, hADAMTS4, hCCN1, hCHTOP, hNDNF) ont été injectés en duplicata à 5 concentrations en série toutes les 300 secondes. Les protéines ont été injectées à 5 concentrations croissantes, à savoir 0,312 nM ; 0,625 nM ; 1,25 nM ; 2,5 nM et 5 nM pour hFGF2 ; 0,625 nM ; 1,25 nM ; 2,5 nM ; 5 nM et 10 nM pour hTFPI ; hCCN1 et hCHTOP et 6,25 nM ; 12,5 nM ; 25 nM ; 50 nM et 100 nM pour hADAMTS4 et hNDNF toutes les 300 s. Ces injections ont été réalisées en utilisant la stratégie Single Cycle Kinetics dans un flux constant d'une solution saline (hepes buffered saline, HBS- EP+, Cytiva) à 30 µL/mL. La protéine hFGF2 connue pour interagir avec l'OTR4132 (données internes) a été utilisée comme contrôle positif car possédant un site de liaison aux héparanes sulfates. La phase de dissociation a été mesurée par injection de la solution saline (HBS-EP+, 10X (Cytiva ref: BR100669) Lot# BCBW6092) pendant 1200 secondes. Entre les duplicatas, les analytes liés à l'OTR4132 ont été éliminés avec du NaCl à 2M (pour hTFPI, hCCN1, hCHTOP et hNDNF) ou du dodécylsulfate de sodium (SDS) à 0,25 % (pour hADAMTS4). L'analyse des données a été réalisée à l'aide du logiciel BiacoreTM Insight Evaluation (Cytiva). Pour chaque protéine, les valeurs des moyennes et l'erreur standard (SD) des constantes d'association (ka), de dissociation (kd) et d'affinité (KD= kd/ka) ou si les valeurs ka et kd ne peuvent pas être déterminé, alors le KD est calculé à partir de la courbe de saturation qui correspond à la valeur de la concentration en abscisse qui est égale à RUmax/2) ont été mesurées. Les résultats obtenus sont représentés sur la figure 5 comprenant (A) les sensogrammes représentent la réponse SPR en fonction du temps pour les 5 protéines et le contrôle positif FGF-2. Les protéines ont été injectées à 5 concentrations croissantes, à savoir 0,312 nM ; 0,625 nM ; 1,25 nM ; 2,5 nM et 5 nM pour hFGF2 ; 0,625 nM ; 1,25 nM ; 2,5 nM ; 5 nM et 10 nM pour hTFPI ; hCCN1 et hCHTOP et 6,25 nM ; 12,5 nM ; 25 nM ; 50 nM et 100 nM pour hADAMTS4 et hNDNF toutes les 300 s. L'interaction a été évaluée en mesurant le ka, le kd et le KD calculé (KD = kd/ka), les résultats obtenus sont représentés dans le Tableau 3 ci-dessous. Les données correspondent au moyenne +/- la déviation standard (SD) des réplicas.

**Tableau 3 : résultats des valeurs de ka, kd et de KD**

| **Protéines** | **k ₐ (1/M.s)** | **SD kₐ (1/M.s)** | **k_{d} (1/s)** | **SD k_{d} (1/s)** | **K_{D} (M)** | **SD K_{D} (M)** |
|---|---|---|---|---|---|---|
| **hFGF2** | 1,45×10⁰⁷ | 2,00×10°⁷ | 1,80×10⁻⁰¹ | 2,54×10⁻⁰¹ | 8,98×10⁻⁰⁹ | 8,24×10⁻⁰⁹ |
| **hCHTOP** | 1,41×10⁰⁷ | 1,77×10⁰⁷ | 7,77×10⁻⁰⁵ | 1,08×10⁻⁰⁴ | 3,35×10⁻¹² | 3,73×10⁻¹² |
| **hTFPI** | 1,20×10⁰⁷ | 1,63×10⁰⁷ | 3,42×10⁻⁰¹ | 4,80×10⁻⁰¹ | 2,29×10⁻⁰⁸ | 2,52×10⁻⁰⁸ |
| **hADAMTS4** | 8,51×10⁰⁵ | 1,19×10⁰⁶ | 1,82×10⁻⁰³ | 2,57×10⁻⁰³ | 1,11×10⁻⁰⁹ | 1,52×10⁻⁰⁹ |

| **Protéines** | **kₐ (1/M.s)** | **SD k ₐ (1/M.s)** | **k_{d} (1/s)** | **SD k_{d} (1/s)** | **K_{D} (M)** | **SD K_{D} (M)** |
|---|---|---|---|---|---|---|
| **hCCN1** | n.d. | n.d. | n.d. | n.d. | n.d. | n.d. |
| **hNDNF** | 3,17×10¹⁰ | 4,48×10¹⁰ | 9,35 | 1,32×10⁻⁰¹ | 6,78×10⁻¹⁰ | 5,40×10⁻¹⁰ |

Dans le tableau, n.d. signifie non déterminé.

La figure 5 l'affinité mesurée desdites protéines à un exemple de polymère biocompatible, par exemple OTR4132. A titre de comparateur, l'affinité du FGF2 humain recombinant pour les RGTA est donnée sous forme de constante d'affinité (KD) de 8,24 nM (8,24 ×10⁻⁹). Tel que démontré sur la figure 5, l'affinité la plus forte a été déterminée pour la protéine hCcn1 (KD non mesurable), et les affinités mesurées pour les 4 autres protéines sont très fortes, à savoir 3,73 ×10⁻¹² pour hCHTOP, 2,52 ×10⁻⁸ pour hTFPI, 1,52 ×10⁻⁹ pour hADAMTS4 et 5,40 ×10⁻¹⁰ pour hNDNF démontrant clairement une liaison entre lesdites protéines et le polymère biocompatible, par exemple l'OTR4132.

Ces résultats démontrent donc clairement que le polymère biocompatible, par exemple l'OTR4132, de part une fixation forte aux protéines sur les vésicules extracellulaires démontre un mécanisme universel de réparation et régénération tissulaire par lequel le polymère biocompatible, par exemple l'OTR4132, permet une vectorisation des vésicules extracellulaires pour atteindre les zones qui disposeraient de sites accessibles de fixation des polymères biocompatibles, par exemple l'OTR4132. En particulier, il est connu que des polymères biocompatibles, par exemple l'OTR4132, se fixe sur des sites dont les héparanes sulfates endogènes ont été dégradés lors de lésions tissulaires, notamment par des glycanases, et remplacent les héparanes sulfates endogènes dégradés lors de lésions tissulaires, et ne sont pas dégradés par les glycanases. Ainsi cet exemple démontre clairement que la combinaison de vésicules extracellulaires et de polymère biocompatible selon l'invention, notamment de part une fixation forte d'un exemple de polymère biocompatible, à savoir l'OTR4132, aux protéines sur les vésicules extracellulaires permet de cibler lesdites vésicules extracellulaires sur des sites lésionnels.

Cet exemple démontre clairement que la combinaison de polymère biocompatible et de vésicules extracellulaires permet avantageusement une vectorisation desdites vésicules extracellulaires sur le site lésionnel quelque soit l'origine cellulaire desdites vésicules extracellulaires. En particulier, cet exemple démontre clairement que le polymère biocompatible se fixe sur les vésicules extracellulaires et permet avantageusement de protéger des glycanases lesdites vésicules et/ou protéines sur lesquelles le polymère biocompatible est fixé. Cet exemple démontre également clairement que la combinaison de polymère biocompatible et de vésicules extracellulaires permet avantageusement un ciblage des lésions tissulaires, par exemple par fixation sur les sites de liaison aux héparanes sulfates.

### Exemple 2: Traitement de lésions tissulaires avec une composition pharmaceutique comprenant un polymère biocompatible et des vésicules extracellulaire

Dans cet exemple, la lésion était une lésion au niveau de cerveau de rat par ischémie selon le procédé décrit dans Khelif et al., 2018, DOI: 10.7150/thno.28252 [28].

Dans cet exemple, le polymère biocompatible était le OTR4132 commercialisé par la société OTR3 Paris France. Des cellules souches mésenchymateuses (MSC) provenant de moelle osseuse de fémurs et tibias de rats Sprague Dawley faisant de 300 à 350 grammes ont été utilisés. Les cellules MSC ont été pré-marquées au bromodeoxyuridine (BrdU, 3 mg / mL, Sigma) par ajout quotidiennement dans le milieu de cuture des MSC, à savoir, pendant 3 jours ([52], Jin, et al., 10.1155/2016/8616143) ([53], Jiang, et al., 10.1007/s13770-021-00421-5). Les cellules ainsi obtenues et marquées ont été injectées par voie intra-veineuse (i.v) à savoir 3×10⁶ cellules dans 300 µl de PBS1X (Référence fournisseur 11503387, Gibco^{™} PBS, pH de 7,4, Fisher Scientific) seules ou en mélange avec le polymère biocompatible OTR4132. La concentration dudit polymère biocompatible OTR4132 dans la composition injectée était de 1,5 mg/kg dilué dans de l'eau stérile saline à 0.9% pour injection (NaCl 0.9% stérile solution for injection, COOPER; Ref: 340093509484; Sérum physiologique, OMNIA, Ref: 32.E0001.

Une analyse histochimique afin d'identifier et quantifier le BrdU au niveau du cerveau entier a été réalisée deux jours après induction de l'ischémie cérébrale. Les coupes ont été lavées dans du PBS1X, incubées dans du HCl 2N à 37°C pendant 1 h, suivies d'une incubation de 10 minutes dans de l'acide borique 0, 1 M. Les coupes ont ensuite été bloquées dans 3 % de BSA (bovine sérum albumine, Thermo Scientific^{™} Blocker^{™} BSA 10% Ref : 1799236, dilué au tiers dans du PBS1X) pendant 2 h à température ambiante, a savoir 20 °C et colorées avec un anticorps polyclonal de rat anti-BrdU (Roche 0,2 µg/mL, Roche 0,2 µg/mL, Cell Prolifération ELISA, BrdU (colorimetric), Ref : 11 647 229 001) pendant une nuit à 4°C. Les coupes ont ensuite été lavées avec du PBS1X (au moins 3 fois, Gibco^{™} PBS, pH de 7,4, FisherScientific Ref : 11503387, suivies d'une incubation de 2 h avec l'anticorps secondaire Alexa Fluor@ 488 (4 µg/mL, Invitrogen) et le marqueur nucléaire Hoechst 33342 (1/5000, Sigma Aldrich) à température ambiante à savoir 22 °C.

Les images ont été obtenues avec un microscope optique (Leica microsystems DMi8). Tous les traitements et analyses d'images ont été effectués à l'aide du logiciel ImageJ^{®} (Wayne Rasband, NIMH, Maryland, USA) (Schneider, Rasband et Eliceiri, 2012). La figure 6 représente les résultats obtenus. Tel que démontré sur la figure 6, des regroupements de MSC sont retrouvés d'une manière prédominante dans l'hémisphère ipsilatéral proche du site lésionnel Figure 6A and 6B. La combinaison du polymère biocompatible OTR4132 avec les MSC augmente très significativement le nombre de MSC localisé dans l'hémisphère ipsilatéral proche du site lésionnel comparé à l'administration de cellules seules (Figure 6B and 6C; Student t-test; p-value= 0.0059).

### Liste de références

[1]. US8790631. Use of biocompatible polymers for the préparation of a composition or a medical device.
[2]. WO2020151900. Cosmetic/dermatological composition
[3]. Barritault, D., Gilbert-Sirieix, M., Rice, K. L., Sineriz, F., Papy-Garcia, D., Baudouin, C., Desgranges, P., Zakine, G., Saffar, J. L., and van Neck, J. RGTA((R)) or ReGeneraTing Agents mimic heparan sulfate in regenerative medicine: from concept to curing patients. Glycoconj J. 2017. 34. 10.1007/s10719-016-9744-5
[4]. Barritault, D., Desgranges, P., Meddahi-Pelle, A., Denoix, J. M., and Saffar, J. L. RGTA((R))-based matrix therapy - A new branch of regenerative medicine in locomotion. Joint Bone Spine. 2017. 84. 10.1016/j.jbspin.2016.06.012
[5]. EP3302523B1. Composition for the treatment of tissue lésions 2016*.*
[6]. Meddahi, A., Bree, F., Papy-Garcia, D., Gautron, J., Barritault, D., and Caruelle, J. P. Pharmacological studies of RGTA(11), a heparan sulfate mimetic polymer, efficient on muscle régénération. J Biomed Mater Res. 2002. 62. 10.1002/jbm.10283
[7]. Pereira, P. M., Papy-Garcia, D., Barritault, D., Chiappini, F., Jackisch, R., Schimchowitsch, S., and Cassel, J. C. Protective Effects of a synthetic glycosaminoglycan mimetic (OTR4132) in a rat immunotoxic lésion model of septohippocampal cholinergic degeneration. Glycoconj J. 2022. 39. 10.1007/s10719-022-10047-x
[8]. Xin, H., Li, Y., Cui, Y., Yang, J. J., Zhang, Z. G., and Chopp, M. Systemic administration of exosomes released from mesenchymal stromal cells promote functional recovery and neurovascular plasticity after stroke in rats. J Cereb Blood Flow Metab. 2013. 33. 10.1038/jcbfm.2013.152
[9]. Doeppner, T. R., Herz, J., Gorgens, A., Schlechter, J., Ludwig, A. K., Radtke, S., de Miroschedji, K., Horn, P. A., Giebel, B., and Hermann, D. M. Extracellular Vesicles Improve Post-Stroke Neuroregeneration and Prevent Postischemic Immunosuppression. Stem Cells Transl Med. 2015. 4. 10.5966/sctm.2015-0078
[10]. Jeyaram, A., and Jay, S. M. Préservation and Storage Stability of Extracellular Vesicles for Therapeutic Applications. AAPS J. 2017. 20. 10.1208/s 12248-017-0160-y
[11]. Ma, Z. J., Yang, J. J., Lu, Y. B., Liu, Z. Y., and Wang, X. X. Mesenchymal stem cell-derived exosomes: Toward cell-free therapeutic strategies in regenerative medicine. World J Stem Cells. 2020. 12. 10.4252/wjsc.v12.i8.814
[12]. Yanez-Mo, M., Siljander, P. R., Andreu, Z., Zavec, A. B., Borras, F. E., Buzas, E. I., Buzas, K., Casal, E., Cappello, F., Carvalho, J., Colas, E., Cordeiro-da Silva, A., Fais, S., Falcon-Perez, J. M., Ghobrial, I. M., Giebel, B., Gimona, M., Graner, M., Gursel, I., Gursel, M., Heegaard, N. H., Hendrix, A., Kierulf, P., Kokubun, K., Kosanovic, M., Kralj-Iglic, V., Kramer-Albers, E. M., Laitinen, S., Lasser, C., Lener, T., Ligeti, E., Line, A., Lipps, G., Llorente, A., Lotvall, J., Mancek-Keber, M., Marcilla, A., Mittelbrunn, M., Nazarenko, I., Nolte-'t Hoen, E. N., Nyman, T. A., O'Driscoll, L., Olivan, M., Oliveira, C., Pallinger, E., Del Portillo, H. A., Reventos, J., Rigau, M., Rohde, E., Sammar, M., Sanchez-Madrid, F., Santarem, N., Schallmoser, K., Ostenfeld, M. S., Stoorvogel, W., Stukelj, R., Van der Grein, S. G., Vasconcelos, M. H., Wauben, M. H., and De Wever, O. Biological properties of extracellular vesicles and their physiological functions. J Extracell Vesicles. 2015. 4. 10.3402/jev.v4.27066
[13]. Hallal, S., Tuzesi, A., Grau, G. E., Buckland, M. E., and Alexander, K. L. Understanding the extracellular vesicle surface for clinical molecular biology. J Extracell Vesicles. 2022. 11. 10.1002/jev2.12260
[14]. Buzas, E. I., Toth, E. A., Sodar, B. W., and Szabo-Taylor, K. E. Molecular interactions at the surface of extracellular vesicles. Semin Immunopathol. 2018. 40. 10.1007/s00281-018-0682-0
[15]. Kalimuthu, K., Kwon, W. Y., and Park, K. S. A simple approach for rapid and cost-effective quantification of extracellular vesicles using a fluorescence polarization technique. J Biol Eng. 2019. 13. 10.1186/s13036-019-0160-9
[16]. Uddin, M. J., Mohite, P., Munde, S., Ade, N., Oladosu, T. A., Chidrawar, V. R., Patel, R., Bhattacharya, S., Paliwal, H., and Singh, S. Extracellular vesicles: The future of therapeutics and drug delivery systems. Intelligent Pharmacy. 2024. 2. 10.1016/j.ipha.2024.02.004
[17]. Fields, G. B. Introduction to peptide synthesis. Curr Protoc Protein Sci. 2002. Chapter 18. 10.1002/0471140864.ps1801s26
[18]. Chan, W. C., and White, P. D. (2000) Fmoc solid phase peptide synthesis : a practical approach, Oxford University Press, New York^0199637253 (hb)
   0199637245 (pbk.)
[19]. Benoiton, L. N. (2005) Chemistry of Peptide Synthesis 9780429119583
[20]. Sanlaville, C., Charmot, D., Petsko, G. A., and Ringe, D. (2008) Structure et fonction des protéines, De Boek University9782804158880
[21]. Raposo, G., and Stoorvogel, W. Extracellular vesicles: exosomes, microvesicles, and friends. J Cell Biol. 2013. 200. 10.1083/jcb.201211138
[22]. Boireau, W., and Elie-Caille, C. Les vésicules extracellulaires. Med Sci (Paris). 2021. 37. 10.1051/medsci/2021201
[23]. Battistelli, M., and Falcieri, E. Apoptotic Bodies: Particular Extracellular Vesicles Involved in Intercellular Communication. Biology (Basel). 2020. 9. 10.3390/biology9010021
[24]. Tan, Z. L., Li, J. F., Luo, H. M., Liu, Y. Y., and Jin, Y. Plant extracellular vesicles: A novel bioactive nanoparticle for tumor therapy. Front Pharmacol. 2022. 13. 10.3389/fphar.2022.1006299
[25]. Takahashi, K., and Yamanaka, S. A decade of transcription factor-mediated reprogramming to pluripotency. Nat Rev Mol Cell Biol. 2016. 17. 10.1038/nrm.2016.8
[26]. Maury, Y., Gauthier, M., Peschanski, M., and Martinat, C. [Human pluripotent stem cells: opening key for pathological modeling]. Med Sci (Paris). 2011. 27. 10.1051/medsci/2011274023
[27]. Caldwell, E. E., Nadkarni, V. D., Fromm, J. R., Linhardt, R. J., and Weiler, J. M. Importance of spécifie amino acids in protein binding sites for heparin and heparan sulfate. Int J Biochem Cell Biol. 1996. 28. 10.1016/1357-2725(95)00123-9
[28]. Hileman, R. E., Fromm, J. R., Weiler, J. M., and Linhardt, R. J. Glycosaminoglycan-protein interactions: définition of consensus sites in glycosaminoglycan binding proteins. BioEssays. 1998. 20. 10.1002/(SICI)1521-1878(199802)20-2<156::AID-BIES8>3.0.CO;2-R
[29]. Wu, H.-f., Lundblad, R. L., and Church, F. C. Neutralization of Heparin Activity by Neutrophil Lactoferrin. Blood. 1995. 85. 10.1182/blood.V85.2.421.421
[30]. Andersson, E., Rydengard, V., Sonesson, A., Morgelin, M., Bjorck, L., and Schmidtchen, A. Antimicrobial activities of heparin-binding peptides. Eur J Biochem. 2004. 271. 10.1111/j.1432-1033.2004.04035.x
[31]. Sobel, M., Soler, D. F., Kermode, J. C., and Harris, R. B. Localization and characterization of a heparin binding domain peptide of human von Willebrand factor. Journal of Biological Chemistry. 1992. 267. 10.1016/S0021-9258(19)50359-3
[32]. Capila, I., and Linhardt, R. J. Heparin-Protein Interactions. Angewandte Chemie International Edition. 2002. 41. 10.1002/1521-3773(20020201)41:3<390::AID-ANIE390>3.0.CO;2-B
[33]. Munoz, E. M., and Linhardt, R. J. Heparin-binding domains in vascular biology. Arterioscler Thromb Vase Biol. 2004. 24. 10.1161/01.ATV.0000137189.22999.3f
[34]. UniProt, C. UniProt: the Universal Protein Knowledgebase in 2023. Nucleic Acids Res. 2023. 51. 10.1093/nar/gkac1052
[35]. Akbar, A., Malekian, F., Baghban, N., Kodam, S. P., and Ullah, M. Méthodologies to Isolate and Purify Clinical Grade Extracellular Vesicles for Medical Applications. Cells. 2022. 11. 10.3390/cells11020186
[36]. Clos-Sansalvador, M., Monguio-Tortajada, M., Roura, S., Franquesa, M., and Borras, F. E. Commonly used methods for extracellular vesicles' enrichment: Implications in downstream analyses and use. Eur J Cell Biol. 2022. 101. 10.1016/j.ejcb.2022.151227
[37]. Liangsupree, T., Multia, E., and Riekkola, M. L. Modem isolation and séparation techniques for extracellular vesicles. J Chromatogr A. 2021. 1636. 10.1016/j.chroma.2020.461773
[38]. Brennan, K., Martin, K., FitzGerald, S. P., O'Sullivan, J., Wu, Y., Blanco, A., Richardson, C., and Mc Gee, M. M. A comparison of methods for the isolation and séparation of extracellular vesicles from protein and lipid particles in human sérum. Scientific Reports. 2020. 10. 10.1038/s41598-020-57497-7
[39]. Panigrahi, A. R., Srinivas, L., and Panda, J. Exosomes: Insights and therapeutic applications in cancer. Transl Oncol. 2022. 21. 10.1016/j.tranon.2022.101439
[40]. Xu, N., Shao, Y., Ye, K., Qu, Y., Memet, O., He, D., and Shen, J. Mesenchymal stem cell-derived exosomes attenuate phosgene-induced acute lung injury in rats. Inhal Toxicol. 2019. 31. 10.1080/08958378.2019.1597220
[41]. Thery, C., Amigorena, S., Raposo, G., and Clayton, A. Isolation and characterization of exosomes from cell culture supernatants and biological fluids. Curr Protoc Cell Biol. 2006. Chapter 3. 10.1002/0471143030.cb0322s30
[42]. Frescaline, G., Bouderlique, T., Mansoor, L., Carpentier, G., Baroukh, B., Sineriz, F., Trouillas, M., Saffar, J. L., Courty, J., Lataillade, J. J., Papy-Garcia, D., and Albanese, P. Glycosaminoglycan mimetic associated to human mesenchymal stem cell-based scaffolds inhibit ectopic bone formation, but induce angiogenesis in vivo. Tissue Eng Part A. 2013. 19. 10.1089/ten.TEA.2012.0377
[43]. Jacquet-Guibon, S., Dupays, A. G., Coudry, V., Crevier-Denoix, N., Leroy, S., Sineriz, F., Chiappini, F., Barritault, D., and Denoix, J. M. Randomized controlled trial demonstrates the benefit of RGTA(R) based matrix therapy to treat tendinopathies in racing horses. PLoS One. 2018. 13. 10.1371/journal.pone.0191796
[44]. US06689741. Cosmetic/dermatological composition
[45]. US2014301972A1. Use of biocompatible polymers for the préparation of a composition or a medical device
[46]. Holmes, B. B., DeVos, S. L., Kfoury, N., Li, M., Jacks, R., Yanamandra, K., Ouidja, M. O., Brodsky, F. M., Marasa, J., Bagchi, D. P., Kotzbauer, P. T., Miller, T. M., Papy-Garcia, D., and Diamond, M. I. Heparan sulfate proteoglycans médiate internalization and propagation of spécifie proteopathic seeds. Proc Natl Acad Sci U S A. 2013. 110. 10.1073/pnas.1301440110
[47]. Ouidja, M. O., Petit, E., Kerros, M. E., Ikeda, Y., Morin, C., Carpentier, G., Barritault, D., Brugere-Picoux, J., Deslys, J. P., Adjou, K., and Papy-Garcia, D. Structure-activity studies of heparan mimetic polyanions for anti-prion thérapies. Biochem Biophys Res Commun. 2007. 363. 10.1016/j.bbrc.2007.08.113
[48]. Cox, J., and Mann, M. MaxQuant enables high peptide identification rates, individualized p.p.b.-range mass accuracies and proteome-wide protein quantification. Nat Biotechnol. 2008. 26. 10.1038/nbt.1511
[49]. Mine, S., Yamazaki, T., Miyata, T., Hara, S., and Kato, H. Structural mechanism for heparin-binding of the third Kunitz domain of human tissue factor pathway inhibitor. Biochemistry. 2002. 41. 10.1021/bi011299g
[50]. Chen, N., Chen, C. C., and Lau, L. F. Adhesion of human skin fibroblasts to Cyr61 is mediated through integrin alpha 6beta 1 and cell surface heparan sulfate proteoglycans. J Biol Chem. 2000. 275. 10.1074/jbc.M003040200
[51]. Jones, G. C., and Riley, G. P. ADAMTS proteinases: a multi-domain, multi-functional family with roles in extracellular matrix turnover and arthritis. Arthritis Res Ther. 2005. 7. 10.1186/ar1783
[52]. Jin, H., Xu, T., Chen, Q., Wu, C., Wang, P., Mao, Q., Zhang, S., Shen, J., and Tong, P. The Fate and Distribution of Autologous Bone Marrow Mesenchymal Stem Cells with Intra-Arterial Infusion in Osteonecrosis of the Femoral Head in Dogs. Stem Cells Int. 2016. 2016. 10.1155/2016/8616143
[53]. Jiang, T., Xia, G., Yang, B., Zhang, H. W., Yin, Y. S., Tang, C. W., and Yang, J. H. Application of Bone Marrow Mesenchymal Stem Cells Effectively Eliminates Endotoxemia to Protect Rat from Acute Liver Failure Induced by Thioacetamide. Tissue Eng Regen Med. 2022. 19. 10.1007/s13770-021-00421-5

## Revendications

1. Composition pharmaceutique comprenant
- un polymère biocompatible de formule générale (I) suivante
AaXxYy (I)
dans laquelle :
A représente un monomère,
X représente un groupement R₁COOR₂ ou -R₉(C=O)R₁₀,
Y représente un groupement O ou N-sulfonate et répondant à l'une des formules suivante -R₃OSO₃R₄, -R₅NSO₃R₆, R₇SO₃R₈ dans lesquelles :
R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation M⁺,
R₇ et R₁₀ représente indépendamment une liaison, une chaîne hydrocarbonée aliphatique, éventuellement ramifiée et/ou insaturée,
a représente le nombre de monomères,
x représente le taux de substitution des monomères A par des groupements X,
y représente le taux de substitution des monomères A par des groupements Y, et
- au moins une vésicule extracellulaire.

2. Composition selon la revendication 1, dans laquelle les monomères A identiques ou différents sont choisis parmi les sucres, les esters, les alcools, les acides aminés, les nucléotides, les acides nucléiques, les protéines ou des dérivés de ceux-ci, de préférence les monomères A sont identiques et sont du glucose.

3. Composition selon la revendication 1 ou 2, dans laquelle le nombre de monomère « a » est tel que la masse desdits polymères de formule (I) est supérieure ou égale à 2000 daltons.

4. Composition selon l'une quelconque des revendications précédentes, dans laquelle le taux de substitution « x » est compris entre 10 et 150%.

5. Composition selon l'une quelconque des revendications précédentes, dans laquelle le taux de substitution « y » est compris entre 10 et 170%.

6. Composition pour son utilisation selon l'une quelconque des revendications précédentes, dans laquelle que ledit polymère biocompatible comprend en outre des groupements chimiques fonctionnels Z, différents de X et Y, capables de conférer audit polymère des propriétés biologiques ou physicochimiques supplémentaires.

7. Composition selon la revendication 6, comprenant
- un polymère biocompatible de formule générale (II) suivante
AaXxYyZz (II)
dans laquelle :
A représente un monomère,
X représente un groupement R₁COOR₂ ou -R₉(C=O)R₁₀,
Y représente un groupement O ou N-sulfonate et répondant à l'une des
formules suivante -R₃OSO₃R₄, -R₅NSO₃R₆, R₇SO₃R₈ dans lesquelles :
R₁, R₃, R₅ et R₉ représentent indépendamment une chaîne hydrocarbonée aliphatique, éventuellement ramifiée et/ou insaturée et qui contient éventuellement un ou plusieurs cycles aromatiques à l'exception de la benzylamine et de la benzylamine sulfonate, R₂, R₄, R₆ et R₈ représentent indépendamment un atome d'hydrogène ou un cation M⁺,
R₇ et R₁₀ représente indépendamment une liaison, une chaîne hydrocarbonée aliphatique, éventuellement ramifiée et/ou insaturée,
Z représente un groupement choisi dans le groupe comprenant des acides aminés, des acides gras, des alcools gras, des céramides, ou des dérivés de ceux-ci, ou encore des séquences nucléotidiques d'adressage.
a représente le nombre de monomères,
x représente le taux de substitution des monomères A par des groupements X,
y représente le taux de substitution des monomères A par des groupements Y,
z représente le taux de substitution des monomères A par des groupements Z, et
- au moins une vésicule extracellulaire.

8. Composition pour son utilisation selon la revendication 6 ou 7, dans laquelle le taux de substitution « z » de l'ensemble des monomères A par des groupements Z est compris de 1 à 50%.

9. Composition selon l'une quelconque des revendications 1 à 8, dans laquelle ladite vésicule extracellulaire est une vésicule extracellulaire de cellules eucaryotes et procaryotes.

10. Composition selon l'une quelconque des revendications 1 à 9, dans laquelle ladite vésicule extracellulaire est une vésicule extracellulaire de cellules eucaryotes choisie dans le groupe comprenant des cellules souches adultes, les cellules pluripotentes natives ou induites (iPS), les cellules multipotentes ou unipotentes, et les cellules différentiées.

11. Composition selon la revendication 10, dans laquelle la vésicule extracellulaire est choisie dans le groupe comprenant les exosomes et les corps apoptotiques.

12. Composition selon l'une quelconque des revendications 1 à 11, dans laquelle la vésicule extracellulaire comprend en outre au moins une protéine de liaison aux héparanes choisie dans le groupe comprenant la chromatine cible de prmt1 (« chromatin target of prmt1 » (Chtop)), l'inhibiteur de la voie du facteur de tissue (« tissue factor pathway inhibitor » (Tfpi)), la disintegrine et métalloprotéinase avec un motif 4 de thrombospondine de type 1 ( « disintegrin and metalloproteinase with thrombospondin type 1 motif 4 » Adamts4), la protéine angiogénique riche en cystéine 61 (« cysteine-rich angiogenic protein 61 » (Ccn1)) et le facteur neurotrophique dérivé des neurones (« neuron-derived neurotrophic factor » (Ndnf)).

13. Composition selon l'une quelconque des revendications 1 à 12 pour son utilisation dans la prévention et/ou dans le traitement de lésions tissulaires.

14. Composition pour son utilisation selon la revendication 13 dans laquelle les lésions tissulaires sont choisie dans le groupe comprenant les lésions du système locomoteur, les lésions vasculaires, les lésions cardiaques, les lésions du système nerveux central, les lésions du système nerveux périphérique, les lésions des systèmes sensoriels, les lésions du système respiratoire, les lésions du tractus digestif, les lésions des tissus osseux, les lésions du tissu pancréatique, les lésions du tissu hépatique, les lésions du tissu rénale, les lésions des voies urinaires et/ou les lésions du tissu vaginal.

15. Procédé de préparation de vésicules extracellulaires avec au moins un polymère biocompatible tel que définie dans l'une quelconque des revendications 1 à 14 comprenant les étapes de :
a. culture de cellules dans un milieu M1
b. culture de ladite au moins au moins une cellule obtenue à l'étape a dans un milieu M2 comprenant un polymère biocompatible un polymère biocompatible tel que définie dans l'une quelconque des revendications 1 à 14,
c. Récupération des vésicules extracellulaires obtenues à l'étape b à partir du milieu M2.

16. Procédé de préparation selon la revendication 15 dans laquelle la durée de l'étape a) d'incubation est comprise de 1 minute à 72 heures

17. Procédé de préparation de la composition selon la revendication 15 ou 16 dans laquelle le milieu M2 comprend une concentration dudit polymère biocompatible de 1 picogrammes/mL à 10 mg/mL, de préférence de 0,1 à 100 µg/mL

18. Vésicule extracellulaire susceptible d'être obtenue selon le procédé défini selon l'une quelconque des revendications 15 à17.

19. Vésicule extracellulaire selon la revendication 18 pour son utilisation dans la prévention et/ou dans le traitement d'une lésion tissulaire.

20. Vésicule extracellulaire pour son utilisation selon la revendication 19, dans laquelle la lésion tissulaire est choisi dans le groupe comprenant une lésion du système locomoteur, une lésion vasculaire, une lésion du système lymphatique, une lésion cardiaque, une lésion du système nerveux central, une lésion du système nerveux périphérique, une lésion des systèmes sensoriels, une lésion du système respiratoire supérieur et/ou inférieur, une lésion du tractus digestif, une lésion des tissus osseux, une lésion du tissu pancréatique, une lésion du système ophtalmique, une lésion du tissu hépatique, une lésion du tissu rénal, une lésion du tissu cutané, une lésion des voies urinaires et/ou une lésion du tissu vaginale et/ou une lésion utérine et/ou une lésion pénienne.
